(19) **Europäisches Patentamt / European Patent Office / Office européen des brevets**

(11) **EP 2 051 201 A1**

(12) **EUROPEAN PATENT APPLICATION**
published in accordance with Art. 153(4) EPC

(43) Date of publication:
**22.04.2009 Bulletin 2009/17**

(21) Application number: **07791275.6**

(22) Date of filing: **25.07.2007**

(51) Int Cl.:
**G06Q 50/00** *(2006.01)*      **G06Q 10/00** *(2006.01)*

(86) International application number:
**PCT/JP2007/064559**

(87) International publication number:
**WO 2008/013193 (31.01.2008 Gazette 2008/05)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **28.07.2006   JP 2006206637**
**30.10.2006   JP 2006293954**

(71) Applicants:
• **Wise Solutions Inc.**
  **Sendai-shi, Miyagi 980-0801 (JP)**
• **Kondo, Yoshiaki**
  **Sendai-shi Miyagi 9893204 (JP)**

(72) Inventors:
• **KONDO, Yoshiaki**
  **Sendai-shi**
  **Miyagi 9893204**
  **(JP)**
• **TATE, Masatoshi**
  **Sendai-shi, Miyagi-ken (JP)**

(74) Representative: **HOFFMANN EITLE**
**Patent- und Rechtsanwälte**
**Arabellastrasse 4**
**81925 München (DE)**

(54) **PROJECT INFORMATION DISPLAY DEVICE, PROJECT INFORMATION DISPLAY METHOD, PROJECT INFORMATION DISPLAY PROGRAM, AND ELECTRONIC MEDICAL RECORD INFORMATION DISPLAY DEVICE**

(57)    Provided is an electronic medical record information display device including: a storage unit (30) for storing calendar information, detailed information on medical operation, and electronic medical record information; a time scale generation unit (41) for acquiring calendar information from the storage unit (30) and generating a plurality of time scales of the different time units; an electronic medical record information acquisition unit (42) for acquiring electronic medical record information corresponding to the time scale of the smallest time unit among the generated time scales from the storage unit (30); display screen information generation unit (43) for generating display screen information from the generated time scales and the acquired electronic medical record information; and a display unit (50) having the display screen for displaying the generated display screen information. The electronic medical record information display device can display project information performing project management in a time unit desired by a user by a simple operation.

FIG. 1

**Description**

Technical Field

The present invention relates to aproject information display device, a project information display method, a project information display program, which are used for a project management of various fields, and an electronic medical record information display device.

Background Art

Conventionally, an application for executing a project management is developed and used in various fields.
During this, managing a patient's treatment plan by an electronic medical record in a medical site is executed.
By using this electronic medical record, a storage space of data can be reduced compared with the time of using a medical record of paper, and information on the past about desired patient can be quickly browsed now on the display screen, without doing the operations of turning over the medical record of paper.
In order to browse the patient's information quickly, it becomes possible to apply a passage and a plan of treatment easy to browse by using a tool which summarizes the medical information called a clinical pathway to the calendar, as shown in Japanese Patent Application Laid-Open Publication No. 2003-108661.
Moreover, by using a technology of the description in Japanese Patent Publication No. 2706645 or Japanese Patent Publication No. 2815346, a schedule of variety of information about the patient can understand at a glance, and also a medical staff can be shared to information in regard of treatment, each medical staff can perform input of data, etc. , and the treatment plan can be suitably changed based on these.

発明の開示

Disclosure of the Invention

However, according to the technology described in above-mentioned Japanese Patent Application Laid-Open Publication No. 2003-108661, Japanese Patent Publication No. 2706645 and Japanese Patent Publication No. 2815346, each information is displayed on a frame of a fixed calendar (unit of day or unit of hour), and it cannot correspond to display information on a still more detailed time unit (for example, unit of minute and unit of second), or to display information over the long period of times, such as unit of year, conversely.
For example, in various project management including the treatment plan in medical science, since each act which composes the project is subordinately and exclusively relevant with other acts in many cases, when it has the detailed information in case subordinate and exclusive relation with the other acts is applied only in predetermined time, the display by the unit of minute or the unit of second may be needed to show these information clearly.
Although it can change into the desired display mode, such as the unit of year, the unit of month, and the unit of day, and the information can be displayed by operating the GUI handler displayed on the display screen according to an invention described in Japanese Patent Application Laid-Open Publication No.H11-212700, a technology in which such change operating of display mode could be made still easier is desired.
Therefore, the object of the present invention is to provide a project information display device, a project information display method, a project information display program, and an electronic medical record information display device which can display the project information which executes a project management by the time unit of a user's desire by easy operation.
A project information display device of the present invention for achieving the above-mentioned object is characterized by comprising: a storage unit configured to store calendar information including date information, a plurality of act information which is detailed information for every act performed in a project, and project information composed by act information selected from the plurality of act information and execution time information for which this selected act information are performed; a time scale generation unit configured to acquire the calendar information of a period designated as a display period from the storage unit, and to generate a plurality of time scales of a different time unit; a project information acquisition unit configured to acquire the project information corresponding to a time scale of the smallest time unit among a plurality of time scales generated by the time scale generation unit, and being included the execution time information within the display period, from the storage unit; a display screen information generation unit configured to generate display screen information from the plurality of time scales generated by the time scale generation unit, and the project information acquired in the project information acquisition unit; and a display unit configured to display the display screen information generated by the display screen information generation unit.

Moreover, a project information display method of the present invention is characterized by comprising: a storing step for storing project information composed by calendar information including date information, a plurality of act information which is detailed information for every act performed in a project, and act information selected from the plurality of act information, and execution time information for which this selected act information is performed; a time scale generation step for acquiring the calendar information of a period designated as a display period from the calendar information stored by the storing step, and generating a plurality of time scales of the different time unit; a project information acquiring step for acquiring the project information corresponding to the time scale of a smallest time unit among the plurality of time scales generated by the time scale generation step, and being included the execution time information within the display period, from the project information stored by the storing step; a display screen information generation step for generating display screen information from the plurality of time scales generated by the time scale generation step, and the project information acquired by the project information acquiring step; and a displaying step for displaying the display screen information generated by the display screen information generation step.

Moreover, aproject information display program of the present invention for achieving a plurality of functions to a project information display device is characterized by comprising: a function for storing project information composed by calendar information including date information, a plurality of act information which is detailed information for every act performed in a project, and act information selected from the plurality of act information, and execution time information for which this selected act information is performed; a function for acquiring the calendar information of a period designated as a display period from the stored calendar information, and generating a plurality of time scales of the different time unit; a function for acquiring the project information corresponding to the time scale of the smallest time unit, and being included the execution time information within the display period among the plurality of generated time scales from the storedproject information: a function for generating display screen information from the plurality of generated time scales and the project information corresponding to the time information of the time scale of the acquired smallest time unit; and a function for displaying the generated display screen information.

Moreover, an electronic medical record information display device of the present invention is characterized by comprising: a function for storing project information composed by calendar information including date information, a plurality of act information which is detailed information for every act performed in a project, and act information selected from the plurality of act information, and execution time information for which this selected act information is performed; a function for acquiring the calendar information of a period designated as a display period from the stored calendar information, and generating a plurality of time scales of the different time unit; a function for acquiring the project information corresponding to the time scale of the smallest time unit, and being included the execution time information within the display period among the plurality of generated time scales from the stored project information; a function for generating display screen information from the plurality of generated time scales and the project information corresponding to the time information of the time scale of the acquired smallest time unit; and a function for displaying the generated display screen information.

Therefore, according to the project information display device of the present invention, the project information display method, the project information display program, and the electronic medical record information display device, the project information for performing project management can be displayed by the time unit of the user's desire by easy operation.

Brief Description of the Drawings

[Fig. 1] It is a block diagram showing a configuration of an electronic medical record display device as a project information display device according to one embodiment of the present invention.
[Fig. 2] It is an explanatory diagram showing a structure of COA used with the electronic medical record display device as the project management display device according to one embodiment of the present invention.
[Fig. 3] It is an explanatory diagram showing a position of the information of COA on a time-line displayed on the electronic medical record display device as the project management display device according to one embodiment of the present invention.
[Fig. 4] It is an indicator chart showing a time scale in a parallel time scale mode displayed on the electronic medical record display device as the project management display device according to one embodiment of the present invention.
[Fig. 5] It is an indicator chart showing a time scale in a time travel scale mode displayed on the electronic medical record display device as the project management display device according to one embodiment of the present invention.
[Fig. 6] It is an explanatory diagram showing a time width of the time scale by unit of day, unit of time, and unit of minute which are displayed on per screen, in the electronic medical record display device as the project management display device according to one embodiment of the present invention.

[Fig. 7] It is an indicator chart showing an example of a time scale displayed on the electronic medical record display device as the project management display device according to one embodiment of the present invention.

[Fig. 8] It is an indicator chart showing an example of the time scale displayed on the electronic medical record display device as the project management display device according to one embodiment of the present invention.

[Fig. 9] It is an indicator chart showing an example of the time scale displayed on the electronic medical record display device as the project management display device according to one embodiment of the present invention.

[Fig. 10] It is an indicator chart showing an example of the time scale displayed on the electronic medical record display device as the project management display device according to one embodiment of the present invention.

[Fig. 11] It is an indicator chart showing an example of the time scale displayed on the electronic medical record display device as the project management display device according to one embodiment of the present invention.

[Fig. 12] It is an indicator chart showing an example of the time scale displayed on the electronic medical record display device as the project management display device according to one embodiment of the present invention.

[Fig. 13] It is an indicator chart showing a display screen displayed on the electronic medical record display device as the project management display device according to one embodiment of the present invention.

[Fig. 14] It is a flow chart showing an operation of the electronic medical record display device as the project management display device according to one embodiment of the present invention.

[Fig. 15] It is an indicator chart showing an example of a time scale and electronic medical record information which are displayed on the electronic medical record display device as the project management display device according to one embodiment of the present invention.

[Fig. 16] It is an indicator chart showing an example of a time scale displayed on the electronic medical record display device as the project management display device according to one embodiment of the present invention.

[Fig. 17] It is an indicator chart showing an example of the time scale displayed on the electronic medical record display device as the project management display device according to one embodiment of the present invention.

[Fig. 18] It is an indicator chart showing an example of the time scale displayed on the electronic medical record display device as the project management display device according to one embodiment of the present invention.

[Fig. 19] It is an indicator chart showing an example of the time scale displayed on the electronic medical record display device as the project management display device according to one embodiment of the present invention.

[Fig. 20] It is an indicator chart showing an example of the time scale displayed on the electronic medical record display device as the project management display device according to one embodiment of the present invention.

[Fig. 21] It is an indicator chart showing an example of the time scale displayed on the electronic medical record display device as the project management display device according to one embodiment of the present invention.

[Fig. 22] It is an enlarged drawing showing a window area manager displayed on the electronic medical record display device as the project management display device according to one embodiment of the present invention.

[Fig. 23] It is an indicator chart showing an example of the time scale and the electronic medical record information which are displayed on the electronic medical record display device as the project management display device according to one embodiment of the present invention.

[Fig. 24] It is an indicator chart showing an example of the time scale and the electronic medical record information which are displayed on the electronic medical record display device as the project management display device according to one embodiment of the present invention.

[Fig. 25] It is an indicator chart showing an example of the time scale and the electronic medical record information which are displayed on the electronic medical record display device as the project management display device according to one embodiment of the present invention.

[Fig. 26] It is an indicator chart showing an example of the time scale and the electronic medical record information which are displayed on the electronic medical record display device as the project management display device according to one embodiment of the present invention.

[Fig. 27] It is an indicator chart showing an example of the time scale and the electronic medical record information which are displayed on the electronic medical record display device as the project management display device according to one embodiment of the present invention.

[Fig. 28] It is an indicator chart showing an example of the time scale and the electronic medical record information which are displayed on the electronic medical record display device as the project management display device according to one embodiment of the present invention.

[Fig. 29] It is an indicator chart showing an example of a time scale and a monthly calendar which are displayed on the project management display device according to another embodiment of the present invention.

[Fig. 30] It is an indicator chart showing an example of the time scale and the monthly calendar which are displayed on the project management display device according to another embodiment of the present invention.

Description of the Preferred Embodiment(s)

An electronic medical record display device for displaying a treatment plan of an electronic medical record in a medical field as one embodiment of a project information display device of the present invention will be explained.

In the electronic medical record display device of this embodiment, medical information stored in the electronic medical record is displayed corresponding to a time scale in which the time-line of the various time units is shown.

<Configuration of Electronic Medical Record Display Device as One Embodiment>

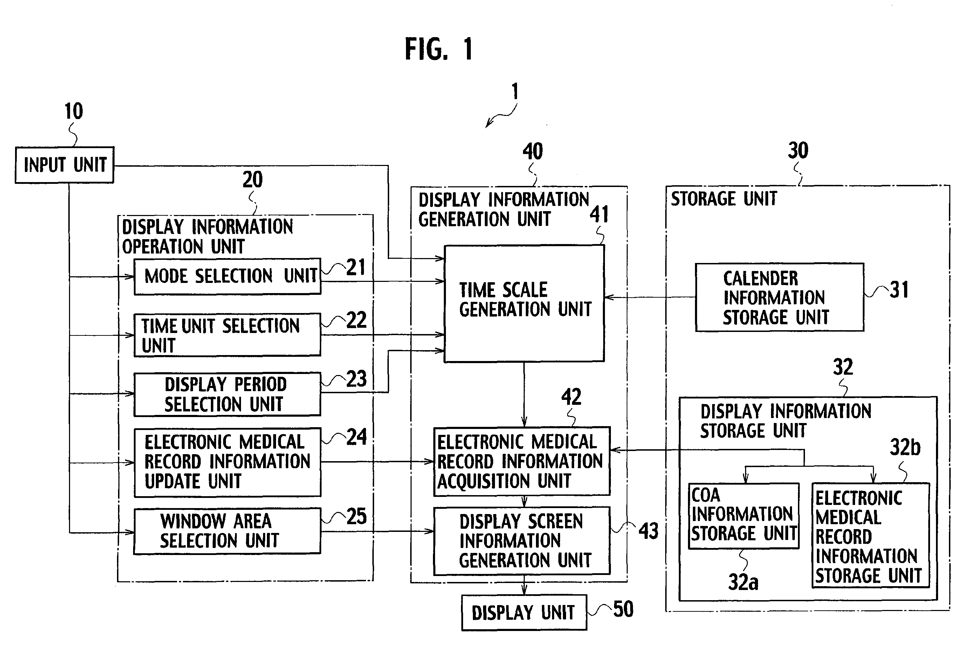

A configuration of an electronic medical record display device 1 in this embodiment will be explained with reference to Fig. 1.

The electronic medical record display device 1 in this embodiment includes an input unit 10, a display information operation unit 20, a storage unit 30, a display information generation unit 40, and a display unit 50.

The input unit 10 is operated by a medical doctor who is a user, and is a mouse, a stylus pen, a keyboard, etc.

The display information operation unit 20 includes a mode selection unit 21, a time unit selection unit 22, a display period selection unit 23, an electronic medical record information update unit 24, and a window area selection unit 25.

The mode selection unit 21 selects a display mode of a time scale displayed on the display screen from a parallel time scale mode and a time travel scale mode which are described later, by operation of the input unit 10 by the medical doctor.

Moreover, the operational mode of the time scale displayed on the display screen and the electronic medical record information is selected from a run time view mode and a snapshot mode which are described later, by operation of the input unit 10 by the medical doctor.

The time unit selection unit 22 selects the predetermined number from unit of decade, unit of year, unit of month, unit of day, unit of hour, unit of minute, or unit of second for the time unit of the time scale displayed on the display screen by operation of the input unit 10 by the medical doctor. The three time units shall be selected in this embodiment.

The display period selection unit 23 selects the display period on which the information is displayed, when the time scale is moved by the medical doctor by using the input unit 10.

The electronic medical record information update unit 24 updates the electronic medical record information displayed on the display screen by operation of the input unit 10 by the medical doctor.

The window area selection unit 25 selects a display area of the display screen information generated by the time scale and electronic medical record information in the display information generation unit 40 by operation of the input unit 10 by the medical doctor.

The storage unit 30 includes a calendar information storage unit 31 and a display information storage unit 32.

The calendar information storage unit 31 stores calendar information including date information for displaying the time scale by the unit of decade, the unit of year, the unit of month, the unit of day, the unit of hour, the unit of minute, or the unit of second.

The display information storage unit 32 includes a COA information storage unit 32a and an electronic medical record information storage unit 32b. The COA information storage unit 32a stores detailed information about the various medical actions inputted beforehand. The electronic medical record information storage unit 32b records electronic medical record information of the unit of decade, the unit of year, the unit of month, the unit of day, the unit of hour, the unit of minute, and the unit of second, comprises information of the medical action selected from information about the medical action stored in the COA information storage unit 32a, and execution time information for which this selected medical action is executed.

The information about the medical action stored in the COA information storage unit 32a will be explained.

In this embodiment, the medical action of various kinds to display is individually defined and managed as an "act".

And each acts of these have the information become the Core of Act (hereinafter, referred to "COA"). The relationship with other acts defined based on the time-line, respectively, etc. are stored in this COA.

There are "observation", "formula", "injection", "supply of food", etc. as a kind of act, and there is information related to other medical actions based on the time-line of having to "observe" after predetermined time passage after "injection", individually, etc.

The information except the medical action, including for example, information of the "symptom", etc., is also COA generated based on the act "observation", and these information (symptom) is held as the COA of "observation". That is, all the medical information is held as the COA of a certain medical action (act).

COA(s) which had the information based on the time-line individually in the electronic medical record display device of this embodiment during the data structure which already had a time-line called the electronic medical record are made to input and display based on such a concept, and providing of high-precision medical information is supported.

- Configuration of COA -

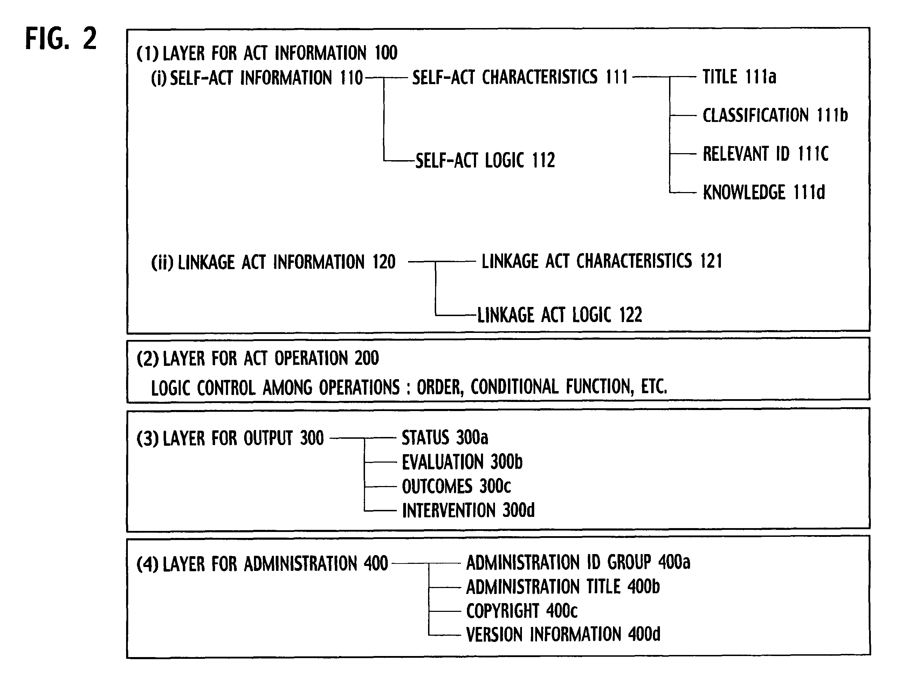

The information which composes each COA used in the present invention will be explained with reference to Fig. 2.

Each COA has (1) layer for act information 100, (2) layer for act operation 200, (3) layer for output 300, and (4) layer for administration 400.

(1) Layer for Act Information 100

The layer for act information 100 has (i) self-act information 110 as self-act information which is information about the self act, and (ii) linkage act information 120 as linkage information which is information for linking with other COA(s).

(i) Self-Act Information 110

The self-act information 110 further includes self-act characteristics 111 as self-act characteristic information, and a self-act logic 112 as self-act logic information. A title 111a (an official title, an another title, a temporary title, etc.), a classification 111b (a main class, a sub class, a minor class, etc.), a relevant ID 111c (discrimination ID for linking to other COA the corresponding to the title 111a), and knowledge 111d (quantity, speed, a unit, scientific knowledge and know-how about the self-act, etc.) of the self act are stored in the self-act characteristics 111.

As an example of the classification 111b, there are "operation act" and "logical on act" as the main class, and there are "observational act" and "interventional act" as the sub class of the "operation act". Moreover, there are "clinical history", "symptom", "finding", etc. as the minor class of the "observational act", and there are the "laboratory test", "image inspection", "physiology inspection", "formula", "injection", "treatment", "admission and discharge", "supply of food" , etc. as the minor class of the "interventional act". Moreover, there are "data generation by the combination of the inspection result", "generation of the prescription; product chit", etc. as the minor class of the "logical on act".

In the self-act logic 112, the information, including a calculating method of the quantity, an appropriate range of the dose, a valuation method of the symptom, unitary conversion, a method of presentation, etc., is stored at the time of performing the self act.

(ii) Linkage Act Information 120

The linkage act information 120 includes linkage act characteristics 121 and linkage act logic 122. In the linkage act characteristics 121, the information, including the content of subordinate and the exclusionary act applied by being linked with other COAs, valid time, the scope, etc. , is stored based on time-line information. In the linkage act logic 122, the conditional function, and the logic flaw, etc., which are such the logical OR, logical AND, at the time of linking with other COAs based on the information of the linkage act characteristics 121 are stored.

(2) Layer for Act Operation 200

In the layer for act operation 200, logic control information among the operations at the time of processing based on the self-act logic 112 and the linkage act logic 122, for example, the execution sequence, the conditional function, etc., are stored.

(3) Layer for Output 300

In the layer for output 300, the information, including a status 300a about the operation of self COA (an assumption, a plan, an operation decision, a preparation, an implementation, a result, etc.), an evaluation 300b (success, failure, etc.), an outcomes 300c (an observation result, an executed result, definition as a graphic, chit and prescription generation, etc.), intervention 300d toward other COA(s), etc., is stored.

(4) Layer for Administration 400

In the layer for administration 400, administration ID group 400a for managing each COA, an administration title 400b, a copyright 400c which applies each COA as works, and version information 400d, such as writing time and the update date, are stored.

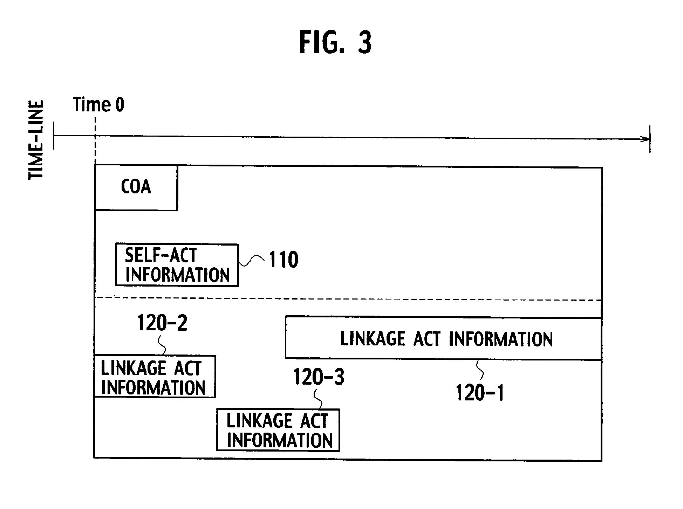

The scope of the information on the time-line of each COA is shown in Fig. 3. The COA can have a plurality of linkage act information 120 respectively. In the COA of Fig. 3, the COA has three linkage acts of the linkage act information 120-1 to 120-3.

In Fig. 3, the linkage act information 120-1 is the information applied after the predetermined time passage after executing medical action according to the self-act information 110, the linkage act information 120-2 is the information applied simultaneously with executing medical action according to the self-act information 110. The linkage act information 120-3 is the information applied from the time of the termination of the medical action according to the self-act information 110.

Returning to Fig. 1, the display information generation unit 40 includes a time scale generation unit 41, an electronic medical record information acquisition unit 42, and a display screen information generation unit 43.

The time scale generation unit 41 acquires the calendar information of the three time units corresponding from the calendar information storage unit 31 of the storage unit 30 based on the information acquired from the input unit 10 or the display information operation unit 20, and generates each time scale. A pointer P is shown at the time which becomes the reference point of the display information in a plurality of generated time scales.

Moreover, the display width of each time unit in the case that this time scale is generated can be freely set as the width which is easy to use if needed.

The time scale to generate includes a parallel time scale mode and a time travel scale mode as the display mode, and is switched by instructions of the mode selection unit 21 of the display information operation unit 20.

The parallel time scale mode is a mode for displaying several time scales with which the time units differ, with the same time width.

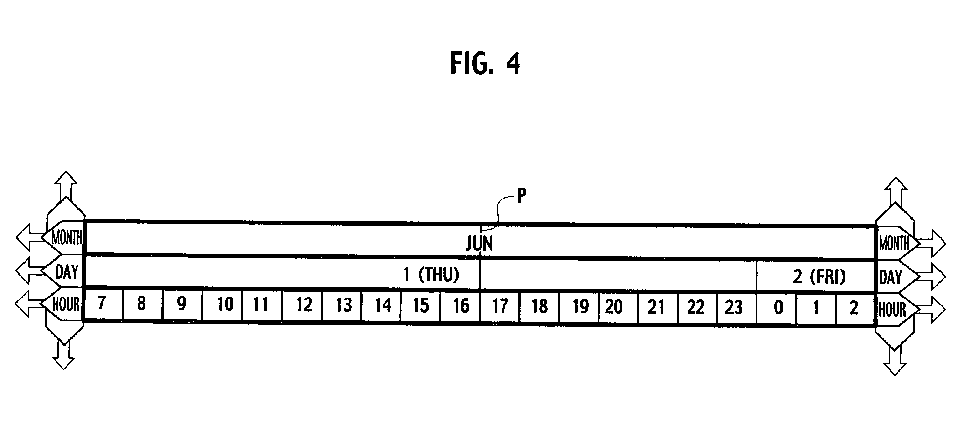

Fig. 4 shows an example which displays the time scale of the three time units, such as the unit of month, the unit of day, and the unit of hour, in the parallel time scale mode. The time frame for the 24 hours of 0 to 23 of the hour unit is expressed as the same width as the time frame for the one day of the unit of day, and the time frame for the 30 days of the 1st to 30th (it changes according to months) of the unit of day is expressed as the same width as the time frame for the one month (in Fig. 2, it is June) of the unit of month.

Thus, since the three time scales may move at the same speed toward the pointer P in which the reference point of display information is shown by displaying the time scale in the parallel time scale mode, when moving any one time scale manually, other time scales are automatically moved at the same speed, and it is updated and displayed so that corresponding date and time may be displayed.

The time travel scale mode is a mode for displaying several time scales with which the time units differ, with different time width.

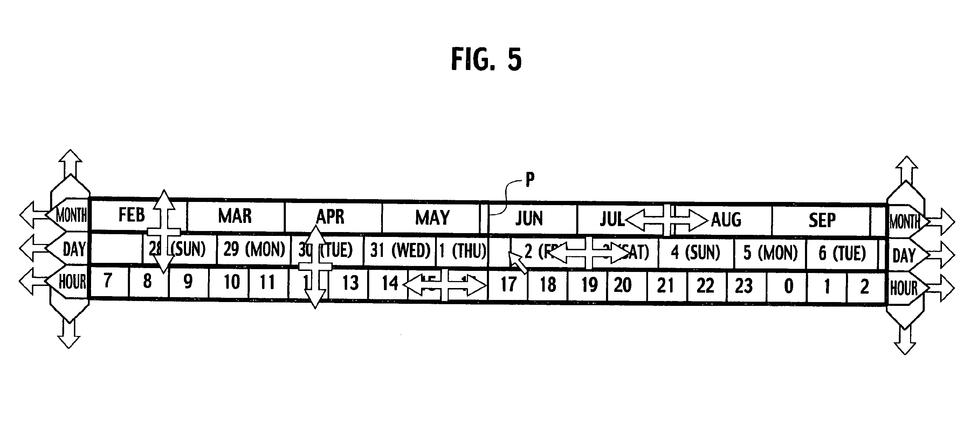

Fig. 5 shows an example which displays the time scale of the three time units, such as the unit of month, the unit of day, and the unit of hour, in the time travel scale mode. The width of the time frame of the unit of day is narrowly set up rather than the time frame of the unit of hour, a plurality of days (in Fig. 3, it is 10 days) are displayed on one screen, and also the width of the time frame of the unit of month is set up narrowly rather than the width of the time frame of the unit of day, and a plurality of months (in Fig. 3, it is 8 months) are displayed on one screen.

Thus, three time scales move at the different speed against the pointer P, which is the reference point of display information by displaying the time scale in the time travel scale mode, and it moves at the late speed, so that the time unit is large in Fig. 5. Therefore, when moving any one time scale manually, other time scales are moved at the automatically different speed and it is updated and displayed so that the corresponding date and time may be displayed.

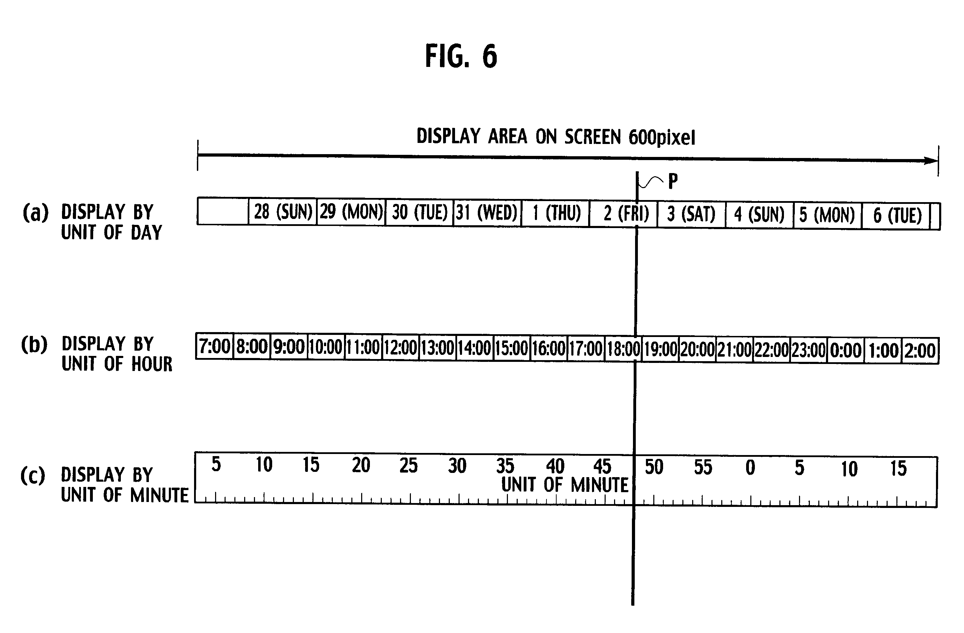

For example, if it sets up display the time frame of the unit of day by the worth on 11 days on the 600 pixels display screen as shown in Fig. 6 (a), the time width per pixel is the 1,584 seconds with the following equation [1].

[Equation 1]

$$11 \text{ day} / 600 \text{ pixels} = (60 \text{ seconds} \times 60 \text{ minutes} \times 24 \text{ hours} \times 11 \text{ days}) / 600 \text{ pixels} = 1,584 \text{ seconds} \qquad \text{Equation [1]}$$

Moreover, if it sets up display the time frame of the unit of hour by the worth on 20 hours on the 600 pixels display screen as shown in Fig. 6 (b), the time width per pixel is the 120 seconds with the following equation [2].

[Equation 2]

$$20 \text{ hours} / 600 \text{ pixels} = (60 \text{ seconds} \times 60 \text{ minutes} \times 20 \text{ hours}) / 600 \text{ pixels} = 120 \text{ seconds} \qquad \text{Equation [2]}$$

Moreover, if it sets up display the time frame of the unit of minute by the worth on 76 minutes on the 600 pixels display screen as shown in Fig. 6 (c), the time width per pixel is the 7.6 seconds with the following equation [3].

[Equation 3]

$$76 \text{ minutes} / 600 \text{ pixels} = (60 \text{ seconds} \times 76 \text{ minutes}) / 600 \text{ pixels} = 7.6 \text{ seconds} \qquad \text{Equation [3]}$$

Thus, since the time width corresponding to 1 pixel differs with time units, when the time scale for the 1 hour is moved in the 1 second, for example on the display screen where the three time scales of Fig. 6 (a) to (c) are displayed simultaneously, the traveling speed of the time scale of the unit of day (a) is only 2 pixels per second, the traveling speed of the time scale of the unit of hour (b) is 30 pixels per second, the traveling speed of the time scale of unit of minute (c) is 473 pixels per second, and traveling speed differs sharply.

Moreover, the time scale to generate includes a run time view mode and a snapshot mode as the operational mode, and is switched by instructions of the mode selection unit 21 of the display information operation unit 20.

The run time view mode is a mode in which the displayed display screen information is updated with the passage of time.

More particularly, the run time view mode includes a pointer flaw mode in which the pointer P moves with the passage of time on the fixed time scale, and a scale flow mode which the time scale moves against the fixed pointer P.

The snapshot mode is a mode which the displayed display screen information is fixed and is not updated with the passage of time.

Moreover, the time scale generation unit 41 changes the time unit of the time scale to generate with instructions of the time unit selection unit 22 of the display information operation unit 20.

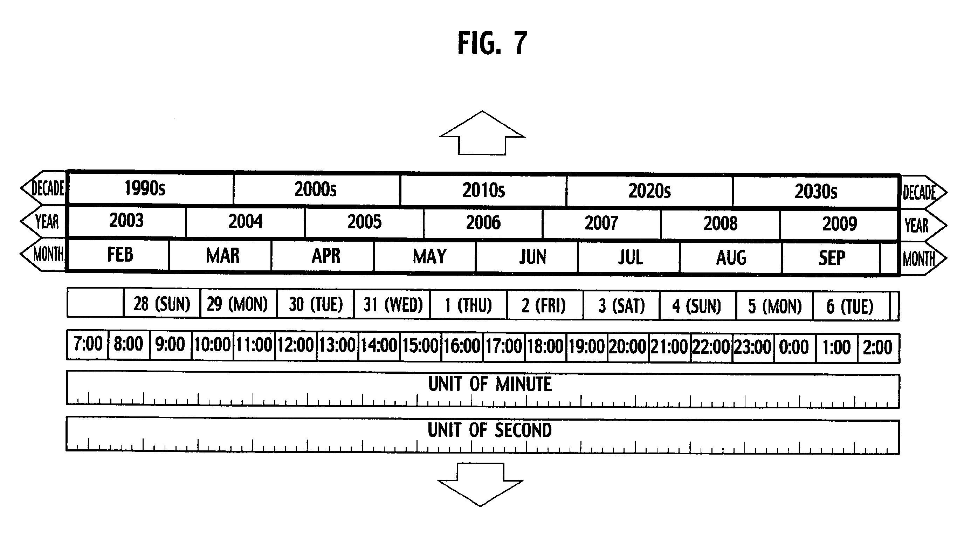

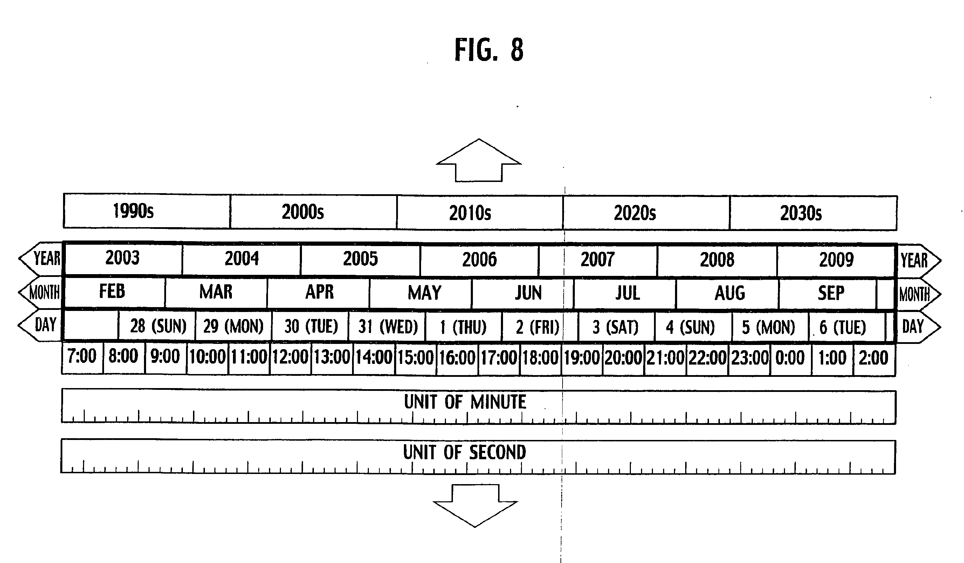

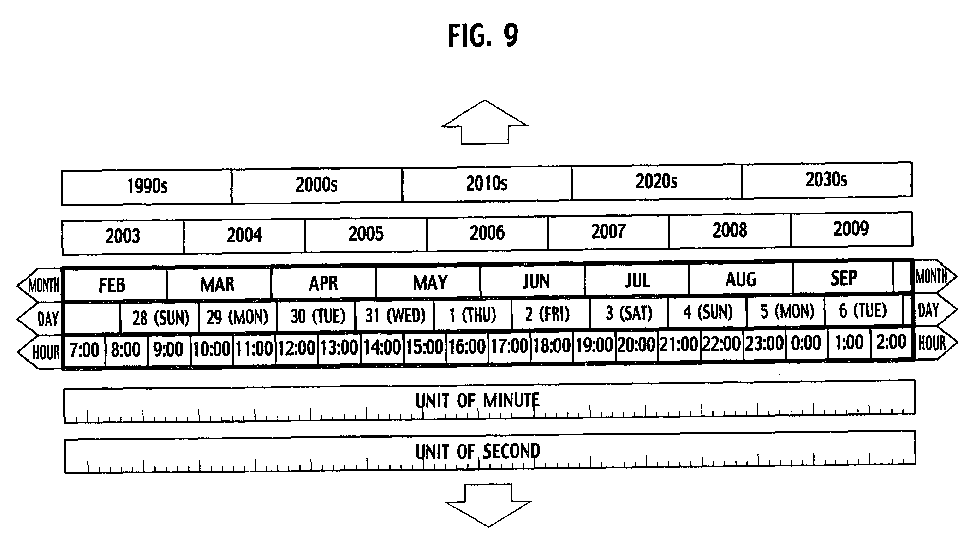

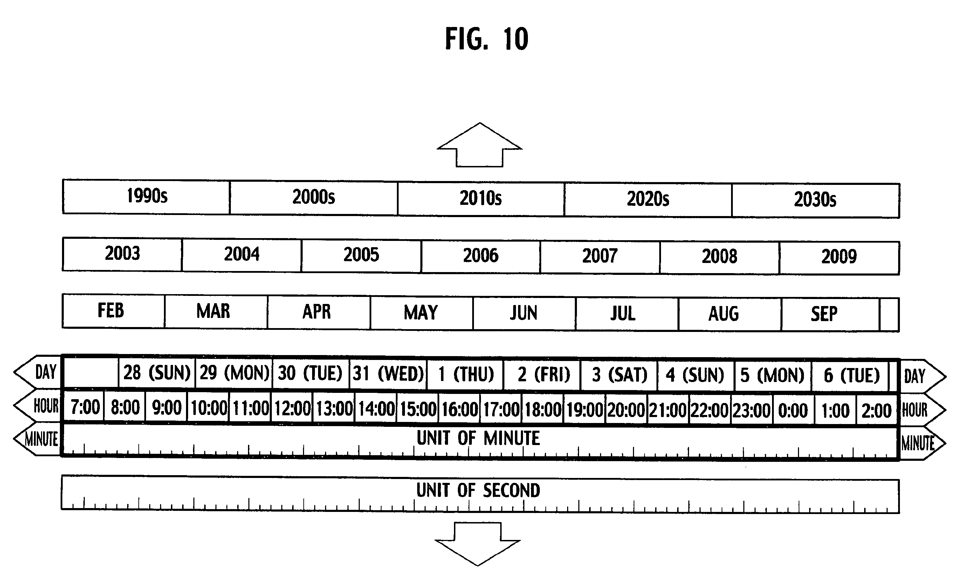

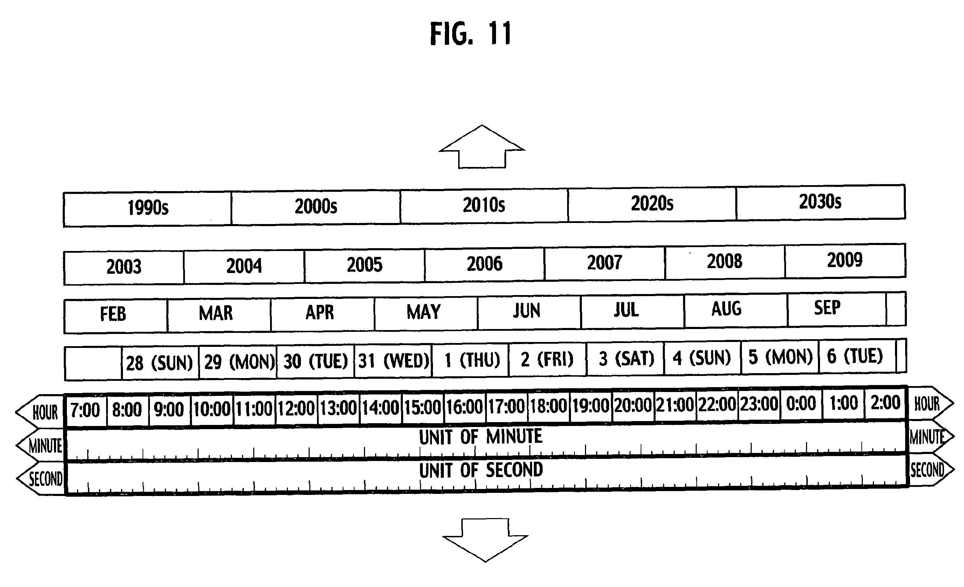

For example, in this embodiment, it can change into, for example, the display by the three time units "(the unit of decade, the unit of year, and the unit of month" as shown in Fig. 7 by the thick line frame, the display by the three time units "the unit of year, the unit of month, and the unit of day" as shown in Fig. 8 by the thick line frame, the display by the three time units "the unit of month, the unit of day, and the unit of hour" as shown in Fig. 9 by the thick line frame, the display by the three time units "the unit of day, the unit of hour, and the unit of minute" as shown in Fig. 10 by the thick line frame, and the display by the three time units "the unit of hour, the unit of minute, and the unit of second" as shown in Fig. 11 by the thick line frame, etc.

Moreover, the time scale generation unit 41 updates the time information of the time scale by moving one of the time scales, or by moving the pointer P, with instructions of the display period selection unit 23 of the display information operation unit 20.

It is switched by operation of a change button (not shown) displayed on an arbitrary position on the display screen whether time information is updated by moving the pointer P or whether time information is updated by moving the time scale.

Based on the time shown by the pointer P of the time scale generated by the time scale generation unit 41, the electronic medical record information acquisition unit 42 acquires the electronic medical record information unit, from the electronic medical record information storage unit 32b of the display information storage unit 32 in the storage unit 30. The acquired electronic medical record is corresponding to the time scale of the unit of hour, which is the smallest time unit among the time scales, and the acquired electronic medical record information is based on COA in which execution time information is included within the display period selected by the display period selection unit 23unitunitunit.

Moreover, the electronic medical record information acquisition unit 42 updates the electronic medical record information already acquired by the instructions from the electronic medical record information update unit 24. When newly adding COA to the electronic medical record, the electronic medical record information is updated by acquiring needed the COA information from the COA information storage unit 32a of the display information storage unit 32 in the storage unit 30.

The display screen information generation unit 43 generates display screen information from the time scale generated by the time scale generation unit 41, the electronic medical record information acquired or updated by the electronic medical record information acquisition unit 42 based on the time shown by the pointer P of this time scale, and the time unit of the time scale, and the window area manager set up beforehand in order to adjust the display area of the electronic medical record information.

The display unit 50 displays the display screen information generated by the display screen information generation unit 43 of the display information generation unit 40, and is a display device etc.

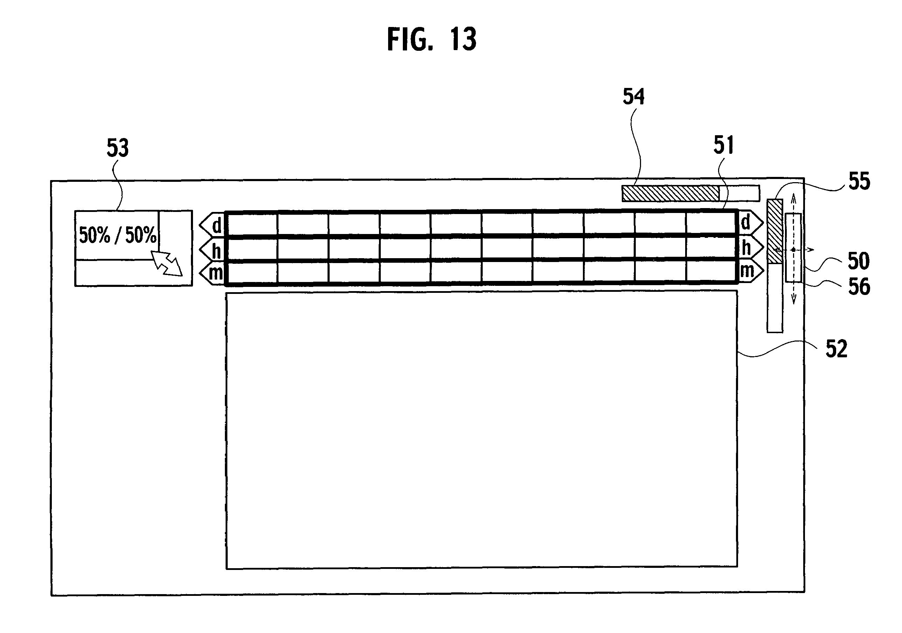

Fig. 13 is an indicator chart showing the status that display screen information is displayed on the display unit 50.

In Fig. 13, the time scale 51 is displayed on the upper part of the screen, and the electronic medical record information 52 corresponding to this time scale 51 is displayed on the lower part of the time scale 51, the window area manager 53 is displayed on the upper part of the screen left, the horizontal time-line window area manager 54 and the vertical time-line window area manager 55 are displayed on the superior extremity of the screen right, and the time-line flexible button 56 is displayed on the arbitrary positions (in this embodiment, it is the top right corner) of the screen.

<Operation of Electronic Medical Record Display Device as One Embodiment>

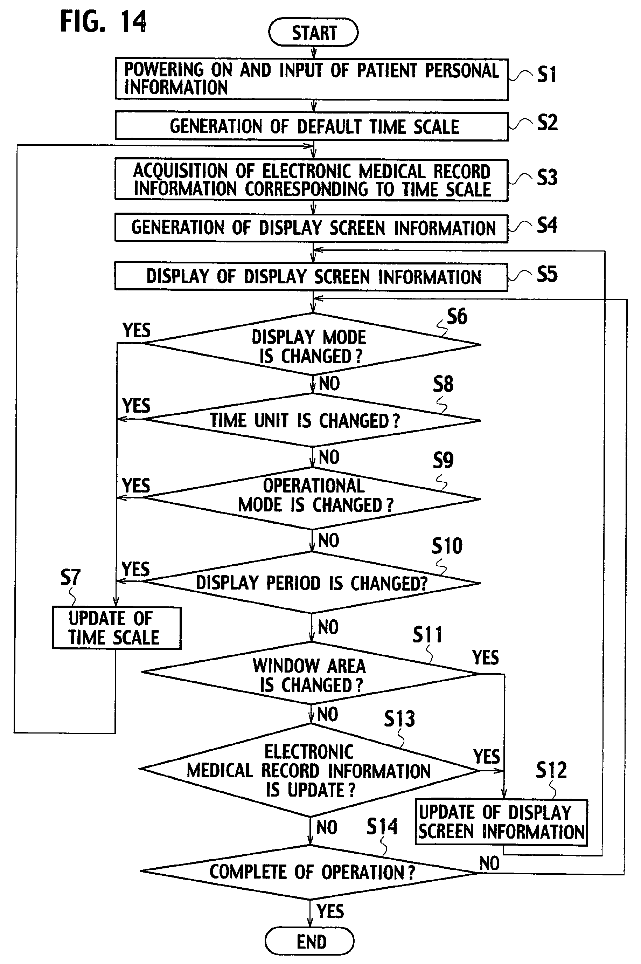

In the following, an operation of the electronic medical record management system 1 as one embodiment will be explained in detail with reference to Fig. 14 to Fig. 28.

As shown in the flow chart of Fig. 14, first of all, when the power supply is supplied to the electronic medical record display device 1 and the desired patient's personal information is inputted by operation of the input unit 10 by the medical doctor (S1), the time scale by the default setup is generated in the time scale generation unit 41 of the display information generation unit 40.

In this embodiment, according to the default configuration of the time scale, as shown in Fig. 4, and the time scale of the three time units, such as the unit of month, the unit of day, and the unit of hour, is generated in the parallel time scale mode (S2). At this point, as for the frame of the unit of hour, which is the minimum time unit, the 20 hours is displayed. Moreover, at this time, the pointer P acting as the reference point of the display information shall be set to the current time at the operation time (17:00 on June 1).

Moreover, as for the time scale according to this embodiment, operational mode is generated to the snapshot mode when the minimum time unit is the unit of decade, the unit of year, the unit of month, the unit of day, or the unit of hour by the default configuration, and generated to the run time view mode when the minimum time unit is the unit of minute or unit of second. Here, since the minimum time unit is the unit of hour, the time scale (7:00 on June 1 to 3:00 on June 2) for the 20 hours on the basis of current time (17:00 on June 1) is generated in snapshot mode.

Next, the electronic medical record information of the unit of hour corresponding to 7:00 on June 1 to 3:00 on June 2 generated with the time scale is acquired from the electronic medical record information storage unit 32b of the display information storage unit 32 in the storage unit 30 by the electronic medical record information acquisition unit 42 (S3).

Next, in the display screen information generation unit 43, display screen information is generated, from the time scale generated by the time scale generation unit 41, and the electronic medical record information acquired by the electronic medical record information acquisition unit 42 (S4).

The generated display screen information is displayed on the display unit 50, as shown in Fig. 13 (S5).

Next, when the time-line of high order time is pointed by operation of the input unit 10 by the medical doctor with the mouse, the pen, etc. , the display mode of the time scale is changed into time travel scale mode from the parallel time scale mode of the default configuration by the mode selection unit 21 (in the case of "YES" in S6), the time scale is updated to the time travel scale mode (S7) as shown in Fig. 5, and it returns to processing of Step S3.

Here, since the current time which the pointer P shows does not change even if the display mode of the time scale changes, the display screen information is generated and displayed from the already acquired electronic medical record information and the updated time scale (S3 to S5).

In the case where the time unit is changed (in the case of "YES" in S8) when changing and displaying the desired display mode or when the transformation of display mode is unnecessary in the state of default configuration (in the case of "NO" in S6), the time unit of the time scale is shifted by operation of the input unit 10 by the medical doctor, and the time scale is updated so that the electronic medical record information of the desired time unit may be displayed (S7).

The time unit of the time scale can be changed into: the display by the three time units "the unit of decade, the unit of year, and the unit of month" as shown in Fig. 7 by the thick line frame; the display by the three time units "the unit of year, the unit of month, and the unit of day" as shown in Fig. 8 by the thick line frame; the display by the three time units "the unit of month, the unit of day, and the unit of hour" as shown in Fig. 9 by the thick line frame; the display by the three time units "the unit of day, the unit of hour, and the unit of minute" as shown in Fig. 10 by the thick line frame; and the display by the three time units "the unit of hour, the unit of minute, and the unit of second" as shown in Fig. 11 by the thick line frame, etc., for example.

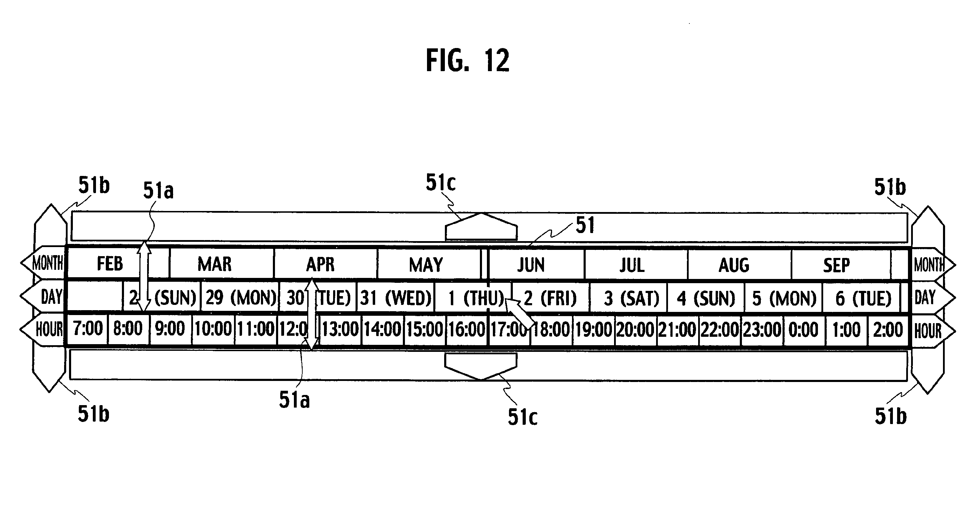

Change of the time unit of the time scale is performed by: the method of moving in the time-lines of the time scale 51 up and down; the method to operate a vertical button 51b of the both ends of the time scale 51; or the method to operate a vertical button 51c placed around the time scale 51, as shown in the arrow 51a of Fig. 12.

Here, as shown in Fig. 11, it is changed into the display by the three time units "the unit of hour, the unit of minute, and the unit of second", and the time scales are updated, and it returns to processing of Step S3.

When returning to Step S3, among the updated time scales, the electronic medical record information corresponding to the unit of second which is the time information of the minimum time unit is acquired from the electronic medical record information storage unit 32b of the display information storage unit 32 in the storage unit 30, and the display screen information is generated and displayed (S3 to S5).

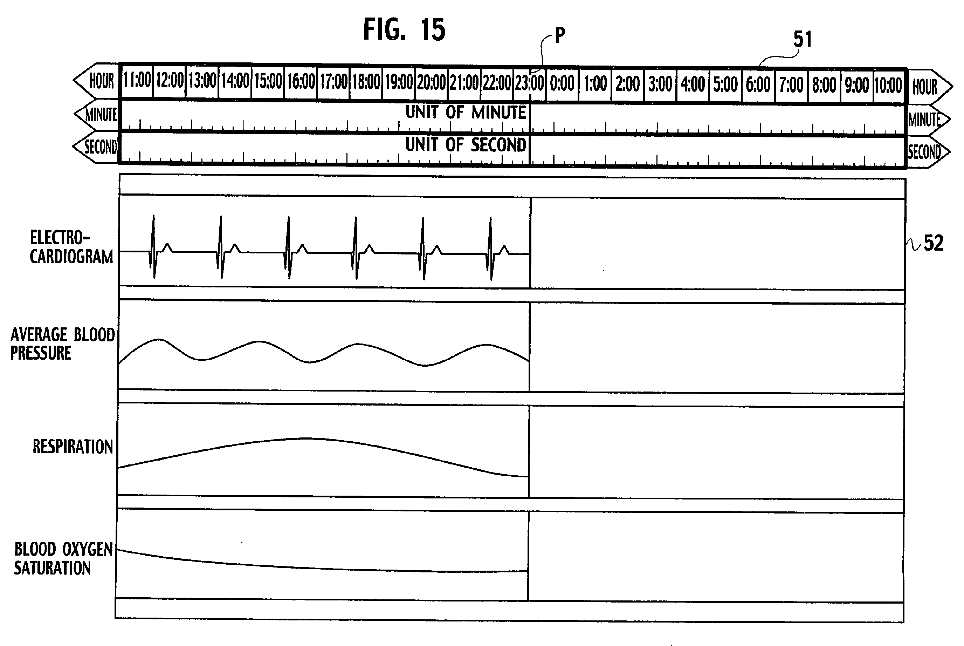

The status that the time scale 51 and the electronic medical record information 52 of the display screen information which are generated by the electronic medical record information corresponding to the second unit are displayed on the display unit 50 is shown in Fig. 15.

Here, since the electronic medical record information 52 of the unit of second is acquired from the electronic medical record information storage unit 32b, as for operational mode, the time scale 51 is generated in the scale flow mode of the run time view mode according to the default configuration.

In the scale flow mode of the run time view mode, the time scale 51 is updated for every per second with passage of time, the electronic medical record information 52 is also acquired, updated, and displayed, with the update of this time scale 51.

In Fig. 15, as for the electronic medical record information 52, the values of the electrocardiogram, average blood pressure, the number of times of respiration, and blood oxygen saturation which are acquired during measurement are displayed in the graphic chart, the horizontal axis is displayed as a time-line, the vertical axis is displayed as a display item, corresponding to the time scale. The new measurement values are further acquired for every per second, and display screen information is updated.

Here, when changing operational mode into the snapshot mode, (in the case of "YES" in S9), it is updated to the fixed time scale 51 by operation of the input unit 10 by the medical doctor, and returns to processing of Step S3.

Here, the display screen information is generated and displayed from the acquired newest electronic medical record information 52 and the updated time scale 51 (S3 to S5).

Next, the case where the display period is changed (in the case of "YES" in S10), when changing into desired operational mode or when change of operational mode is unnecessary (in the case of "NO" in Step S9), will be explained.

Here, the case where the display period is changed will be explained with the three time units of the unit of month, the unit of day, and unit of hour whose the time scale 51 is default configuration.

There are change by the click mode and change by slide mode in the change of the display period.

These click modes and slide mode are further classified into the analog mode and digital mode, respectively.

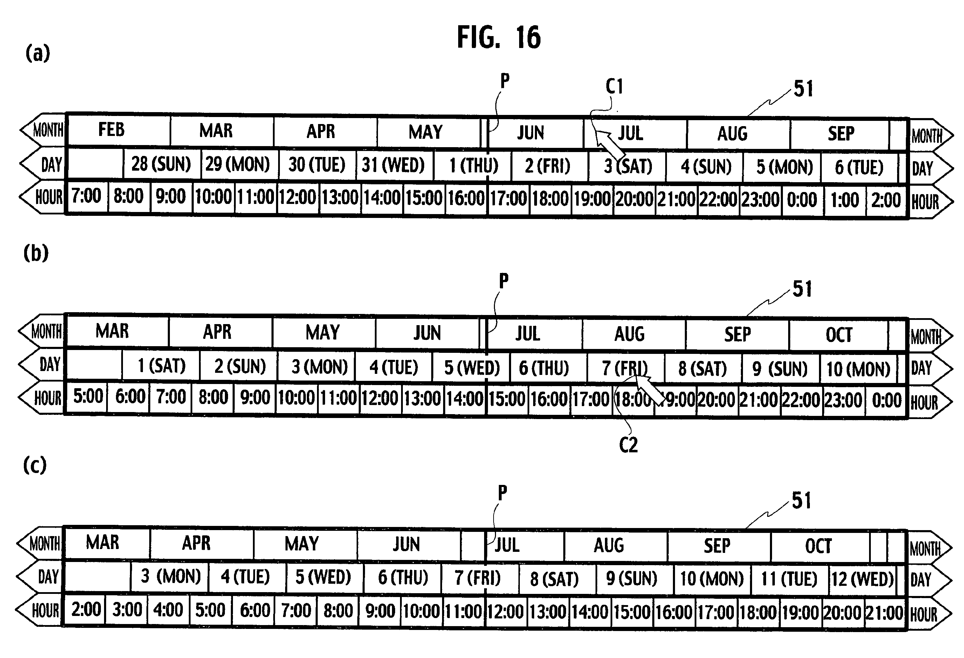

The case where the display period is changed by the analog mode in the click mode will be explained with reference to Fig. 16.

In the analog mode in the click mode, when the input unit 10 is used by the medical doctor and the desired position is clicked on the time scale 51 of the arbitrary time units, all the time-lines under the clicked time unit currently displayed is moved in an instant so that the absolute time shown by the clicked position may adjust to the pointer P.

Therefore, transit time becomes large, so that the position distant from the pointer P is clicked.

Here, as shown in Fig. 16(a), in the status that the pointer P is adjusted to at "17:00 on June 1 (Thursday)", if the position C1 which corresponds to on the time-line of the unit of month at "15:00 on July 5 (Wednesday)" is clicked all the time-lines (the unit of month, the unit of day, the unit of hour) are moved so that the "15:00 on July 5 (Wednesday)" which is the absolute time shown by the clicked position C1 as shown in Fig. 16(b) may be adjusted to the pointer P.

Furthermore, if the position C2 which corresponds to at "12 : 00 on the 7th (Friday)" on the time-line of the unit of day is clicked in the state of Fig. 16(b), all the time-lines (the unit of day, the unit of hour) under the unit of day are moved so that "12:00 on July 7 (Friday)" which is the absolute time shown by the clicked position C2 as shown in Fig. 16(c) may adjust to the pointer P.

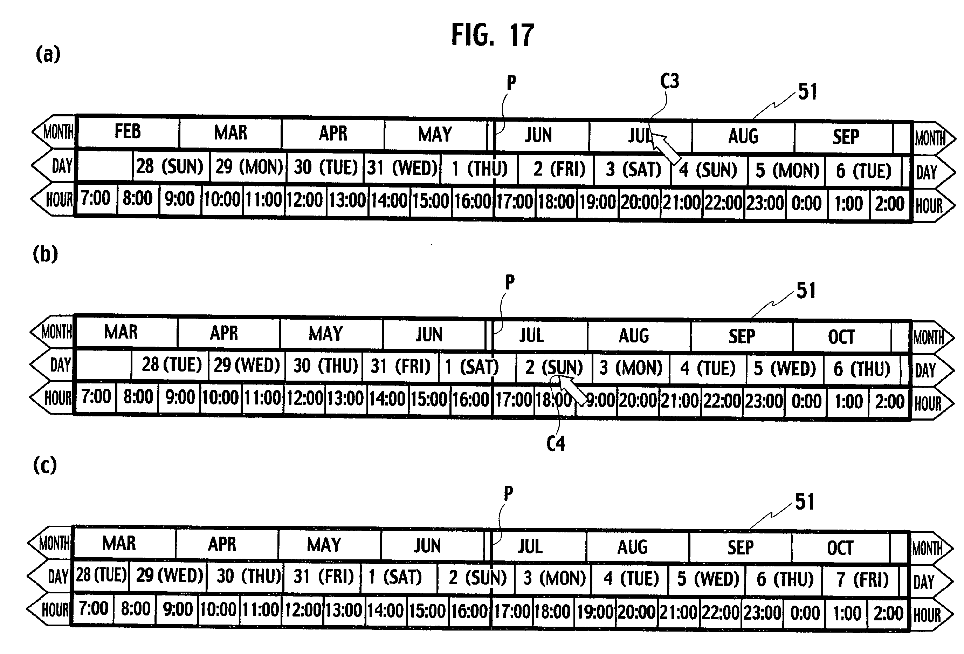

Next, the case where the display period is changed by the digital mode in the click mode will be explained with reference to Fig. 17.

In the digital mode in the click mode, when the input unit 10 is used by the medical doctor and the desired position is clicked on the time scale 51 of the arbitrary time units, the time-line of the time unit smaller than the clicked time unit is fixed, and it is moved so that the absolute time shown by the clicked position may adjust to the pointer P.

Here, as shown in Fig. 17(a), in the status that the pointer P adjusts at "17:00 on June 1 (Thursday)", when the position C3 which corresponds to the arbitrary positions in July on the time-line of the unit of month is clicked the time-line of the unit of day and the unit of hour which is the time unit smaller than the clicked time unit is fixed, the time-line of the unit of month is moved in an instant so that "July" shown by the clicked position C3 may adjust to the pointer P, and the time-line of the unit of month is adjusted by the pointer P at "17:00 on July 1 (Saturday)", as shown in Fig. 16(b).

If the position C4 which corresponds to the arbitrary positions on "2nd (Sunday)" on the time-line of the unit of day in the state of Fig. 17 (b) is further clicked, the time-line of the unit of hour which is the time unit smaller than the clicked time unit is fixed, the time-line of the unit of day is moved in an instant so that "2nd" shown by the clicked position C4 may adjust to the pointer, and the time-line of the day unit is adjusted by the pointer P at "17:00 on July 2 (Sunday)", as shown in Fig. 17(c).

If it is operated, for example on the time-line of the unit of month by changing the display period in the digital mode in this click mode, it can move so that the arbitrary positions of the different month in the same date, hour, minute, and second may be adjusted with the pointer P.

Moreover, in the analog mode in slide mode, the input unit 10 is used by the medical doctor, and if it is slid so that the desired position of the time-line of the arbitrary time units may adjust to the pointer P, it will be moved so that the time-line of all the time units may adjust to the absolute time shown by the position adjusted to the pointer P of the slid time-line.

For example, when adjusting at "23:00 on February 15", in the time-line of the unit of month, first of all, as for the time-line of the unit of day, the day of the neighborhood already centering on "15th" is displayed by sliding so that the pointer P may adjust to near the center of the frame of "February", as for time-line of the unit of hour, the time of the neighborhood already centering on "before 0:00" is displayed by sliding so that the pointer P may adjust to near the right end of the frame on the "15th" in the time-line of this unit of day, and it is adjusted by being slid so that "23: 00" may finally adjust to the pointer P in the time-line of this unit of hour.

Moreover, in the digital mode in slide mode, the input unit 10 is used by the medical doctor, and if it is slid so that the desired position of the time-line of the arbitrary time units may adjust to the pointer P, the time-line of the time unit smaller than the slid time unit is fixed, and only the slid time-line is moved.

The digital mode in this slide mode can also display the one information after another of the different month in the same date, hour, minute, and second by operating, for example, on the time-line of the unit of month, as well as the digital mode in the click mode.

As for this click mode and slide mode, operation by the click mode is performed by clicking the time-line with the left button of the mouse, these modes are used properly by performing operation by slide mode by sliding the time-line, pushing the right button of the mouse, or it changes by operating the mode switching button (not shown) displayed on external of the time scale 51 on the screen by using the mouse or the pen.

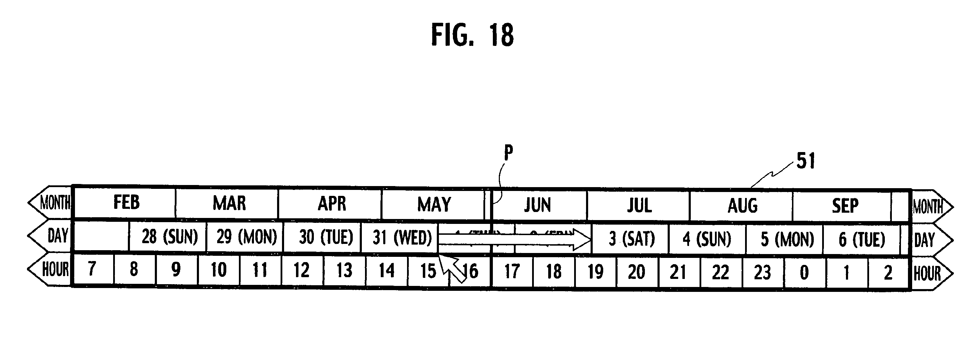

Moreover, a span mode for changing the range of the display period will be explained with reference to Fig. 18 to Fig. 21.

The span mode for changing the range of the display period is a mode which all the display rectangles of the minimum time unit in the time scale are enlarged to the selected time range, and moves to the display by which zoom out is performed, when the desired time range (time span) on the time scale is selected.

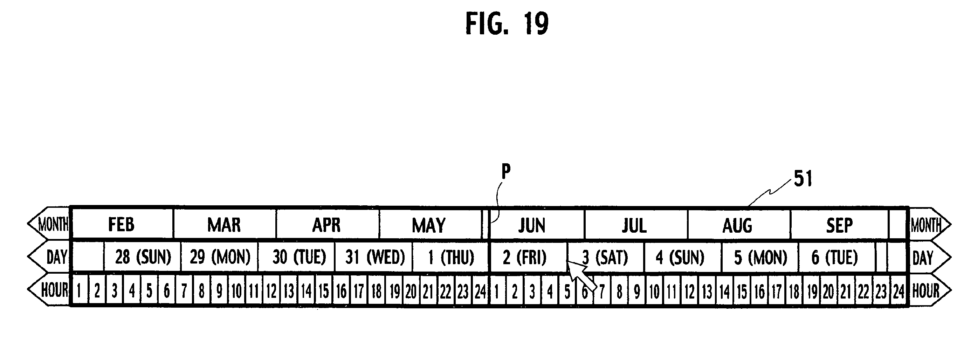

For example, among the time scale 51 displayed by the three time units, such as the unit of month, the unit of day, and the unit of hour, as shown in Fig. 18, when "1st (Thursday) to 2nd (Friday)" is selected from the time-line of the unit of day when drag operation is done by the medical doctor with the cursor, zoom out of all the display rectangles of the unit of hour which is the minimum time unit in this time scale 51 is performed at "Thursday, 1st 0:00 to Friday, 2nd 24:00", and the display rate of the unit of hour is changed, and is displayed as shown in Fig. 19.

At this time, when the display of the unit of hour becomes detailed too much by changing the display rate, the auto-shift may be made to be performed so that the time unit displayed may constitute the time unit of the suitable size.

When the cursor by which drag operation is performed is removed from the time-line of the unit of day, the time scale 51 returns to the parallel time scale mode.

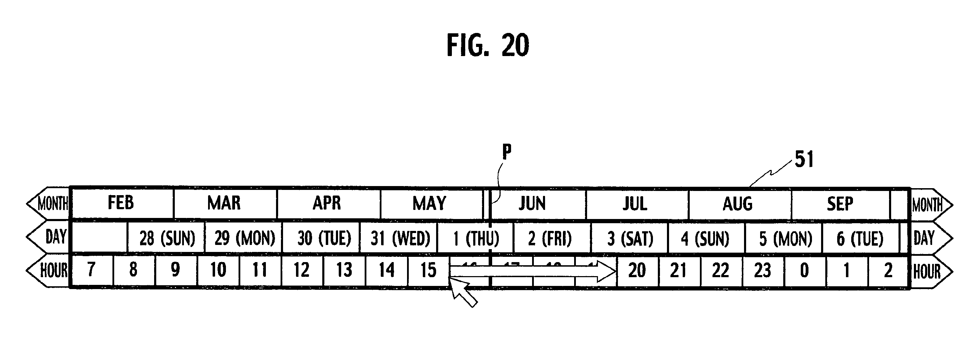

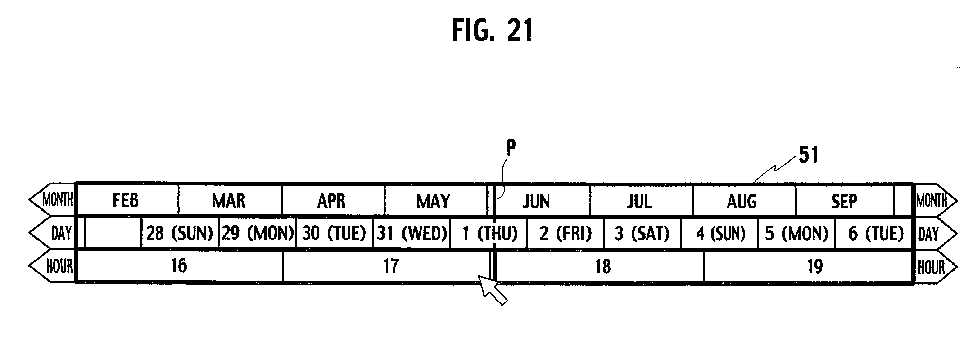

Moreover, among the time scale 51 displayed by the three time units, such as the unit of month, the unit of day, and the unit of hour, as shown in Fig. 20, when "16:00 to 20:00" is selected from the time-line of the unit of hour when drag operation is done by the medical doctor with the cursor, all the display rectangles of the unit of hour which is the minimum time unit in this time scale 51 zoom in "16:00 to 20:00", and the display rate of the unit of hour is changed, and is displayed as shown in Fig. 21.

At this time, when the display of the unit of hour becomes coarse too much by changing the display rate, the auto-shift may be made to be performed so that the time unit displayed may constitute the time unit of the suitable size.

Here, when the cursor by which the drag operation is performed is removed from the time-line of the unit of day, the time scale 51 returns to the parallel time scale mode.

As for the time range (time span) selected by these span modes:

1) The operated drug length may be made to be applied to all the display rectangles of the minimum time unit in that condition; or
2) The operated drug length may be made to be applied to all the display rectangles of the minimum time unit in the units, such as the day, the hour, and the minute to be set up gradually.

In the case where the display is changed with the method (1), for example , when drag operation is performed from the center position of "1st (Thursday)" of the time-line of the unit of day to the position of 2/3 from ahead of "Friday, 2nd" , "Thursday, 1st 12:00 to Friday, 2nd 16:00" is applied to all the display rectangles of the minimum time unit.

Moreover, when the display is changed with the method (2), for example, when drag operation is similarly performed from the center position of "1st (Thu)" of the time-line of the unit of day to the position of 2/3 from ahead of "Friday, the 2nd", drug length is determined by the unit of day and "Thursday, 1st 0:00 to Friday, 2nd 24:00" is applied to all the display rectangles of the minimum time unit.

Themethods (1) and (2) which are mentioned above can be changed and used according to the object.

When the time scale 51 is updated by the operation in this span mode, the electronic medical record information is also updated and displayed with this updating.

Thus, by performing moving operation of the time scale 51, it is changed at the desired display period, the time scale 51 is updated (S7), and it returns to processing of Step S3.

At Step S3, the electronic medical record information 52 corresponding to the time information of the unit of hour of the updated time scale 51 is acquired from the electronic medical record information storage unit 32b, and display screen information is generated and displayed (S3 to S5).

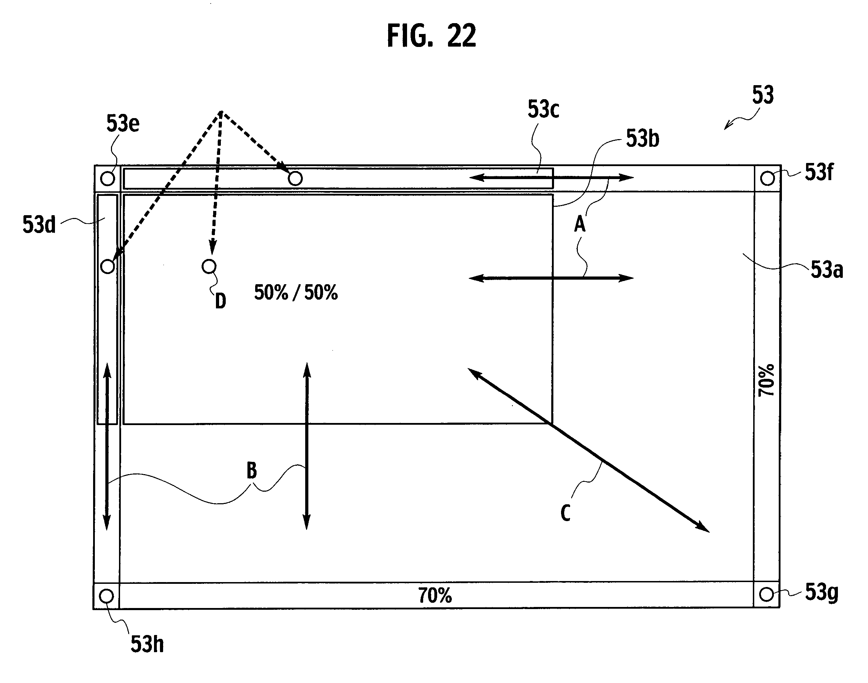

Furthermore, processing when changing a window area of this displayed display screen information (in the case of "YES" in S11) will be explained with reference to Fig. 13 and Fig. 22.

In the status that the display screen information is displayed on the display unit 50, when the input unit 10 is used by the medical doctor. A window area manager 53, a horizontal time-line window area manager 54, a vertical time-line window area manager 55, or a time-line flexible button 56 is operated, the window area displayed on the electronic medical record information 52 is changed (in the case of "YES" in S11).

The window area manager 53 includes: a full-screen display area 53a in the case of displaying all the information acquired from the electronic medical record information storage unit 32b corresponding to the time scale 51; a sector display area 53b for designating the area to enlarge and display within this full-screen display area; a horizontal sidebar 53c for extending or reducing the horizontal display area of this sector display area 53b; a vertical sidebar 53d for extending or reducing the vertical display area of the sector display area 53b; a click button 53e for displaying the horizontal vertical minimum range of the sector display area 53b, a click button 53f for displaying the horizontal maximum range and the vertical minimum range of the sector display area 53b; a click button 53g for displaying the horizontal vertical maximum range of the sector display area 53b; and a click button 53h for displaying the horizontal minimum range and the vertical maximum range of the sector display area 53b, and displays the desired electronic medical record information 52 enlarging or reducing by operating the size of the sector display area 53b.

In this window area manager 53, the electronic medical record information displayed by operating the size of the sector display area 53b is extended or reduced about the horizontal time base direction on the basis of the position of the fixed pointer P on the time scale 51, and is extended or reduced about the number of the display items of vertical electronic medical record information on the basis of the positions (for example, position etc. which shows the superior extremity of full-screen display area a, or the center of the vertical axis) set up by the user.

The sector display area 53b is operated by a drag mode by which the size of the sector display area 53b is changed when the horizontal axis, the vertical axis, the horizontal sidebar 53c, or the vertical sidebar 53d of the sector display area 53b is dragged by the input unit 10, or operated by a click mode by which the size of the display partial area 53b is changed by clicking the arbitrary positions among the sector display area 53b, the horizontal sidebar 53c, or the vertical sidebar 53d.

In the drag mode, the operation to which only the time-line length shown by the horizontal axis is extended or reduced by moving the transversal frame line of the sector display area 53b such as the arrow A of Fig. 22 or by moving the horizontal sidebar 53c, the operation which increases and decreases the number of the display items of COA shown with the vertical axis by moving a closing line under the sector display area 53b such as the arrow B, or by moving the vertical sidebar 53d, and the operation of performing simultaneously extension or reduction of time-line length, and the increase and decrease of the number of the display items in COA by moving the corner of the frame of the sector display area 53b such as the arrow C, are possible

Moreover, in the click mode, the operation of performing simultaneously extension or reduction of time-line length and the increase and decrease of the number of the display items in COA is possible by clicking the arbitrary places among the full-screen display area 53a, the horizontal sidebar 53c, or the vertical sidebar 53d such as the point D of Fig. 22.

Furthermore, in the click mode, the horizontal vertical minimum range of the sector display area 53b is displayed when the click button 53e is clicked, the horizontal maximum range and the vertical minimum range of the sector display area 53b are displayed when either of click buttons 53f to 53h is clicked, the horizontal vertical maximum range of the sector display area 53b is displayed when the click button 53g is clicked, and the horizontal minimum range and the vertical maximum range of the sector display area 53b are displayed when the click button 53h is clicked.

Moreover, the operation to which the display position of the direction of the horizontal axis (time-line) of the sector display area 53b in the full-screen display area 53a is moved can be changed by operating the display area on a horizontal time-line window area manager 54 shown in Fig. 13 by the drag mode.

Moreover, the operation to which the display position of the direction of the vertical axis (display item of COA) of the sector display area 53b in the full-screen display area 53a is moved can be changed by operating the display area on a vertical time-line window area manager 55 shown in Fig. 13 by the drag mode.

Moreover, by setting up so that the minimum display width of the time unit of the high order and the maximal display width of the time unit of the continuous low order may be in agreement between the continuous time units (for example, the unit of hour -> unit of day, the unit of day -> unit of month, etc.), the time unit corresponding to the electronic medical record information to display can be switched continuously by dragging the time-line flexible button 56 to the upper and lower sides or right and left.

For example, the display area is reduced, and the electronic medical record information is elongated and displayed by dragging the time-line flexible button 56 to the top or the right. It changes to the time unit of one low order, and the electronic medical record information is displayed if the drug is further continued even if it reaches the minimum display width of the time unit corresponding to the electronic medical record information on display.

Moreover, the display area is extended, and the electronic medical record information is compressed and displayed by dragging the time-line flexible button 56 to the bottom or the left. It changes to the time unit of one high order, and the electronic medical record information is displayed if the drug is further continued even if it reaches the maximal display width of the time unit corresponding to the electronic medical record information on display.

By performing one of operations among these, the content displayed on the electronic medical record information 52 is updated by the electronic medical record information 52 corresponding to the sector display area 53b after changing (S12), and it returns to processing of Step S3.

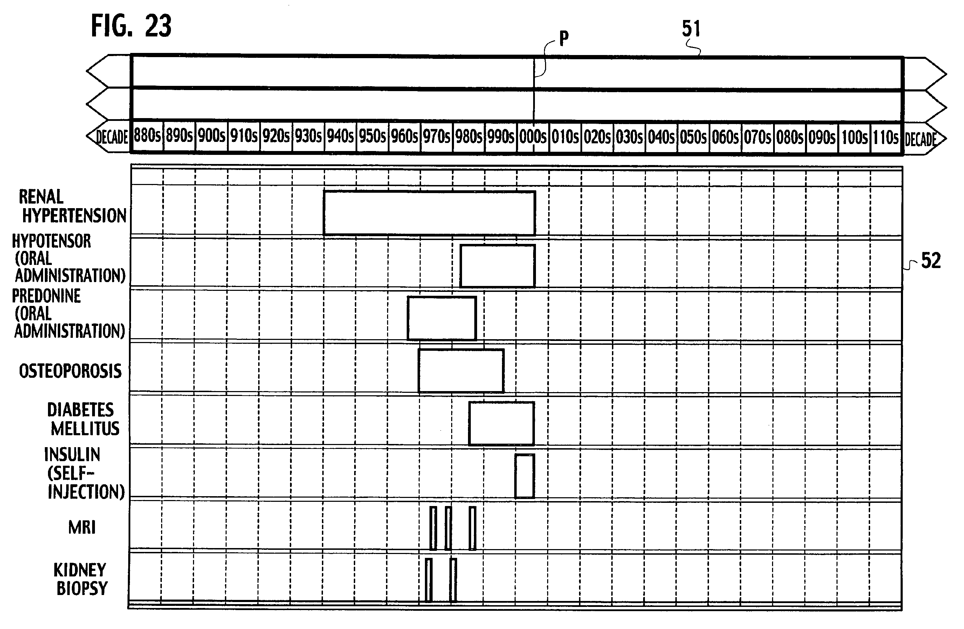

By repeating processing of these Step S6 to Step S12, the example of the time scale 51 and the electronic medical record information 52 which are displayed on the display unit 50 is shown in Fig. 23 to Fig. 28.

Fig. 23 shows the time scale 51 and the electronic medical record information 52 when the time travel scale mode select is applied as the display mode, the time unit of the decadeunit is selected as the time unit of the time scale 51, it is adjusted so that the pointer P may match as the display period in the 2000s (2000 to 2010), and the screen display information is updated and is displayed on the display device 15 so that COA whose the electronic medical record information 52 is the 8 items ("Renal hypertension", "hypotensor (oral administration)", "predonine (oral administration)", "osteoporosis", "diabetes mellitus", "insulin (self-injection)", "MRI", "Kidney biopsy") of the 240 years may be displayed by the window area manager 53.

In Fig. 23, the COA indicates that: the symptom of the renal hypertension is verified between 1940 to 2005; the administration of the hypotensor (oral administration) is performed between 1982 to 2005; the administration of predonine (oral administration) is performed between 1965 to 1985; the symptom of osteoporosis is verified between 1970 to 1995; the diabetic symptom is verified between 1985 to 2005; the insulin (self-injection) is performed between 2000 to 2005; MRI is performed of the 3 times in 1973, 1979, and 1987; and the kidney biopsy is performed of the twice in 1972 and 1980, as the acquired electronic medical record information 52.

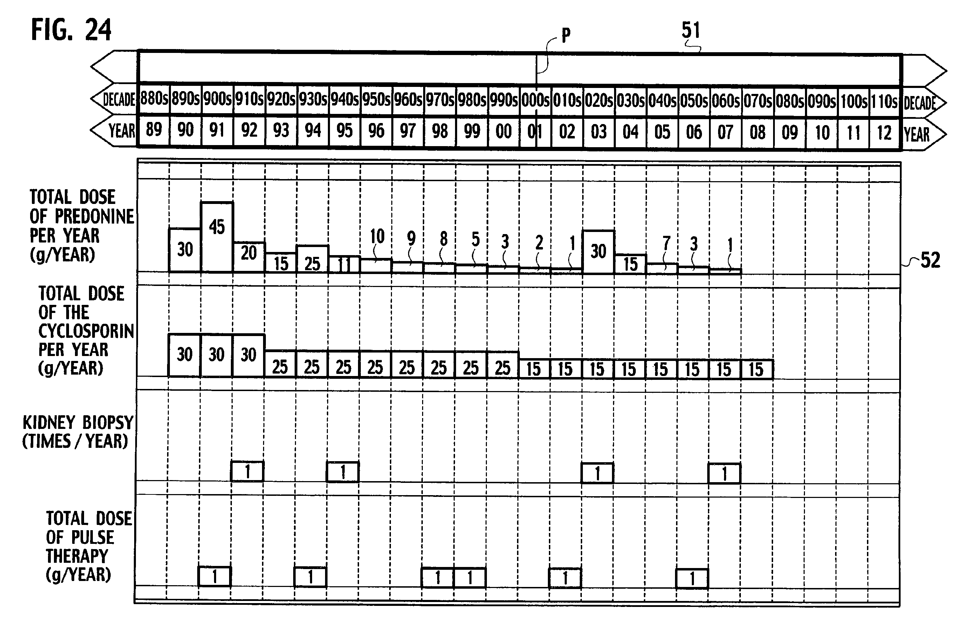

Fig. 24 shows the time scale 51 and the electronic medical record information 52 when screen display information is updated and is displayed on the display device 15 so that: the time travel scale mode select may be applied as the display mode; the two time units, such as the unit of decade and the unit of year, are selected as the time unit of the time scale 51; it is adjusted so that the pointer P may match as the display period in 2001; and the four items ("the predonine total dose per year", "the cyclosporin total dose per year", "the kidney biopsy", "the pulse therapy total dose") of the COA of the 24 years is displayed for the electronic medical record information 52 by the window area manager 53.

In Fig. 24, the COA indicates that: the predonine is dosed with 30g, 45g, 20g, 15g, 25g, 11g, 10g, 9g, 8g, 5g, 3g, 2g, 1g, 30g, 15g, 7g, 3g, and 1g per each year in 1990 to 2007 of the 18 years; the cyclosporin is dosed with 30g, 30g, 30g, 25g, 25g, 25g, 25g, 25g, 25g, 25g, 25g, 15g, 15g, 15g, 15g, 15g, 15g, 15g and 15g per each year in 1990 to 2008 of the 19 years; the kidney biopsy is performed by 4 times in 1992, 1995, 2003, and 2007; and the administration by the pulse therapy is performed at 1g in 1991, at 1g in 1994, at 1g in 1998, at 1g in 1999, at 1g in 2002, and at 1g in 2006, as the acquired electronic medical record information 52.

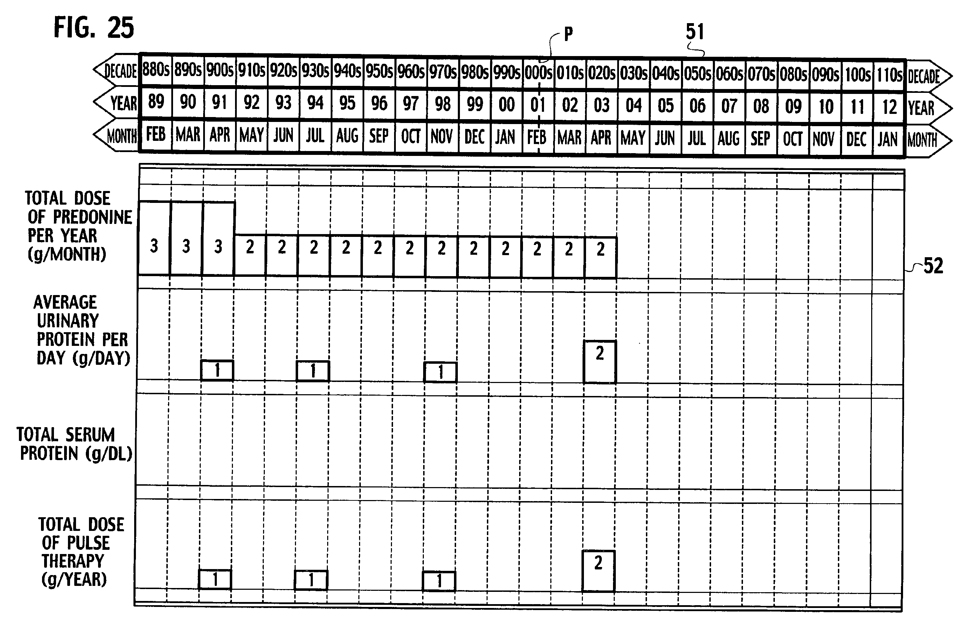

Fig. 25 shows the time scale 51 and the electronic medical record information 52 when screen display information is updated and is displayed on the display device 15 so that: the time travel scale mode is selected as the display mode; the three time units, such as the unit of decade, the unit of year, and the unit of month, are selected as the time unit of the time scale 51; it is adjusted so that the pointer P may match as the display period in February, 2001; and the four items ("the predonine total dose per month", "the average urinary protein per day", "the total serum protein", the "the pulse therapy total dose") of COA for the 24 months is displayed for the electronic medical record information 52 by the window area manager 53.

In Fig. 25, COA indicates that: the predonine is dosed with 3g, 3g, 3g, 2g, 2g, 2g, 2g, 2g, 2g, 2g, 2g, 2g, 2g, 2g, and 2g per every month in February, 2000 to April, 2001 of the 15 months; the average urinary protein per day is detected of 1g in April, 2000, 1g in July, 1g in November, and 2g in April, 2001; the total serum protein is not detected; and the administration by the pulse therapy is performed at 1g in April, 2000, at 1g in July, at 1g in November, and at 2g in April, 2001, as the acquired electronic medical record information 52.

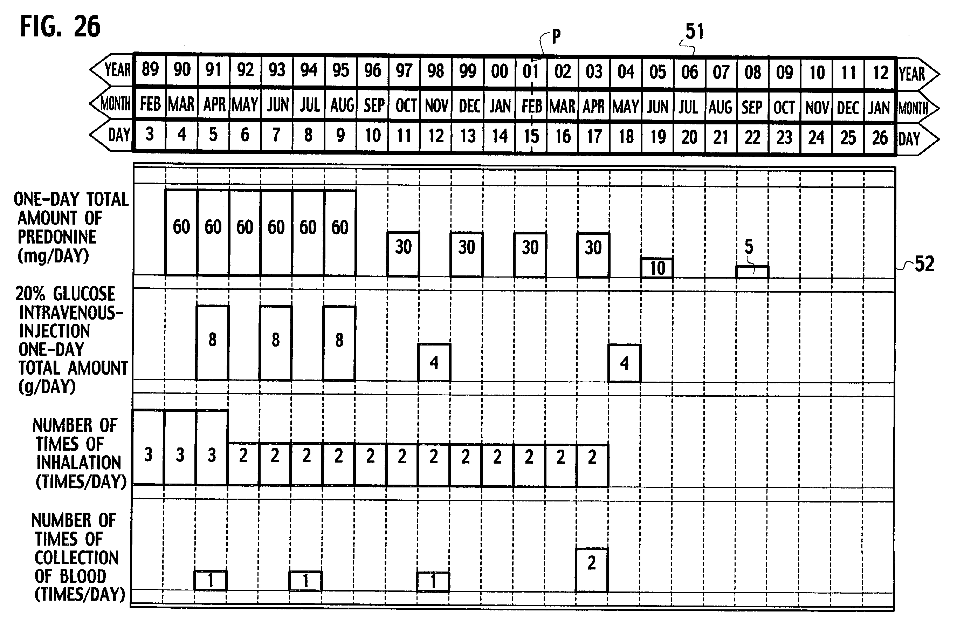

Fig. 26 shows the time scale 51 and the electronic medical record information 52 when the screen display information is updated and is displayed on the display device 15 so that: the time travel scale mode select is applied as the display mode; the three time units, such as the unit of year, the unit of month, and the unit of day, are selected as the time unit of the time scale 51; it is adjusted so that the pointer P may match as the display period on February 15, 2001; and the four items ("the prednisolone one-day total amount", "20% glucose intravenous injection one day total amount", "the number of times of inhalation", "the number of times of collection of blood") of the COA for the 24 days is displayed for the electronic medical record information 52 by the window area manager 53.

In Fig. 26, the COA indicates that the prednisolone is prescribed for the patient at 60 mg per day between February 4, 2001 to February 9, at 30 mg per day on the February 11, February 13, February 15, and February 17, at 10 mg on February 19, and at 5 mg on February 22; the 20% glucose intravenous injection is prescribed for the patient at 8g per day on February 5, 2001, February 7, and February 9, and at 4g per day on February 12 and February 18: the inhalation is performed by 3 times per day in February 3, 2001 to February 5 of the three days, and twice per day in February 6 to February 17 of the 12 days, and the collection of blood is performed at once on February 5, 2001, February 8, and February 12, and twice on February 17, as the acquired electronic medical record information 52.

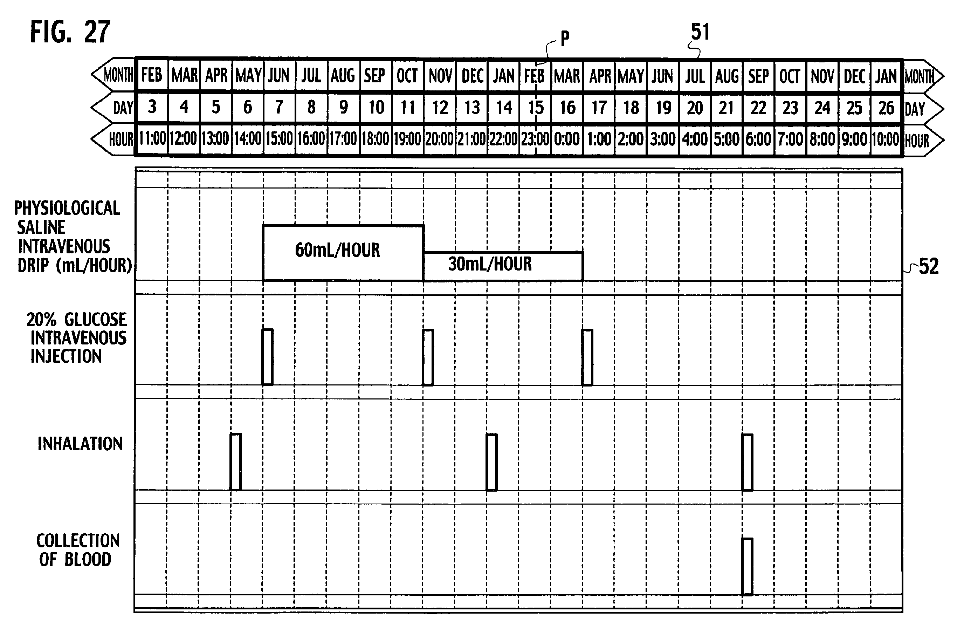

Fig. 27 shows the time scale 51 and the electronic medical record information 52 when the screen display information is updated and is displayed on the display device 15 so that: the time travel scale mode is selected as the display mode; the three time units, such as the unit of month, the unit of day, and the unit of hour, are selected as the time unit of the time scale 51; it is adjusted so that the pointer P may match as the display period at 23:00 on February 15; the four items ("the physiological saline intravenous drip", "the 20% glucose intravenous injection", "the inhalation", "the collection of blood") of the COA for the 24 hours is displayed for the electronic medical record information 52 by the window area manager 53.

In Fig. 27, the COA indicates that: the quantity of 60 mL per hour is prescribed for the patient for the physiological saline intravenous drip from 15:00 to 19:00 on February 15, and also 30 mL per hour is prescribed for the patient from 20:00 on February 15 to 1:00 on February 16; the 20% glucose intravenous injection is performed for 3 times at 15:00 on February 15, 20:00 on February 15, and 1: 00 on February 16; the inhalation is performed for 3 times at 14:00 on February 15, 22:00 on February 15, and 6:00 on February 16; and the collection of blood is performed at 6:00 on February 16, as the acquired electronic medical record information 52.

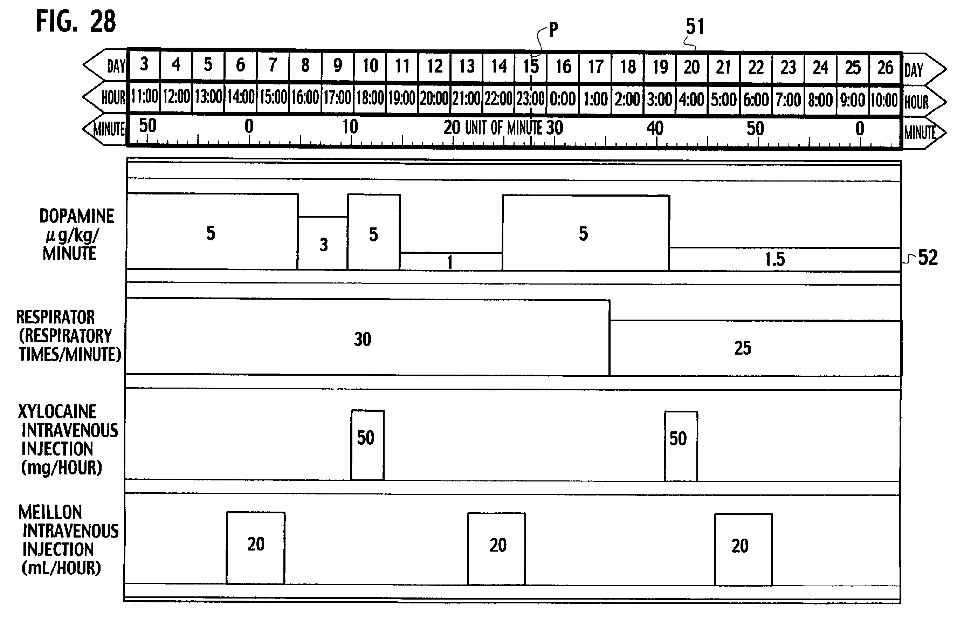

Fig. 28 shows the time scale 51 and the electronic medical record information 52 when the screen display information is updated and is displayed on the display device 15 so that: the time travel scale mode is selected as the display mode; the three time units, such as the unit of day, the unit of hour, and the unit of minute, are selected as the time unit of the time scale 51; it is adjusted so that the pointer P may match as the display period at 23:27 on 15th; and the four items ("the dopamine", "the respirator", "the xylocaine intravenous injection", "the meillon intravenous injection") of the COA for the 76 minutes is displayed for the electronic medical record information 52 by the window area manager 53.

In Fig. 28, COA indicates that: 5 micrograms/kg/minute of the dopamine are prescribed for the patient in the 17 minutes of the beginning for the 76 minutes 22:48 on the 15th to 24:04, and 3 micrograms/kg/minute is prescribed for the patient in the following 4 minutes, 5 micrograms/kg/minute is prescribed for the patient in the following 6 minutes, 1 micrograms/kg/minute is prescribed for the patient in the following 9 minutes, 5 micrograms/kg/minute is prescribed for the patient in the following 17 minutes, and 1.5 micrograms/kg/minute is prescribed for the patient in the following 23 minutes; the number of times of respiration by the respirator is set as per minute 30 times even for 23:35 on the 15th, and 23:35 or later on the 15th are set as per minute 25 times; 50 mg of the xylocaine intravenous injections are prescribed for the patient from 23:10 on the 15th to the 13 minutes, and from 23:41 to 44 minutes, respectively; and 20 mL of the meillon intravenous injections are prescribed for the patient from 22:58 on the 15th to 23:04, from 23:21 to the 27 minutes, and from 23:45 to 51 minutes, respectively, as the acquired electronic medical record information 52.

Whenupdating, adding new COA information to these electronic medical record information 52 displayed on the display units 50 etc. (in the case of "YES" in S12), the desired COA information is acquired from the COA information storage unit 32a of the display information storage unit 32 of the storage unit 14 by the electronic medical record information acquisition unit 42 by operation of the electronic medical record information 52 update unit 24 by the medical doctor using the input unit 10, and the display screen information is updated in the display screen information generation unit 43 (S13).

Processing when inputted into the electronic medical record information 52 which the desired COA information acquired from the COA information storage unit 32a is acquired by the electronic medical record information acquisition unit 42, and is already composed will be explained.

In the COA already composed on the electronic medical record information 52 and the COA newly added, as mentioned above, respectively, the linkage act characteristics 121 including the link information with other COAs, and the linkage act information 120 which is composed of the linkage act logic 122, etc. are stored.

These linkage act characteristics 121 are stored by relative time using the time-line on the basis of the time of operation of each COA, and when the COA information is operated by the input unit 10, the information using these time-lines is displayed.

When adding new COA information to the electronic medical record information acquisition unit 42, the existence or nonexistence of the relevance are detected among the linkage act characteristics 121 stored in this new COA in the time which it is going to add, and the linkage act characteristics 121 stored in COA already inputted into the electronic medical record information 52.

Since the COA already inputted into the electronic medical record information 52 is inputted at the time corresponding to the time scale 51, the absolute time is decided by the information based on the time-line information included in the execution time and the stored linkage act characteristics 121 of this COA.

When the relevance is detected between the COA which it is going to add, and other COAs as a result of detection, processing for displaying a warning screen, processing for adding the COA generated subordinately, processing for deleting the COA which has exclusive relationship, etc. are executed, and the display screen information is updated.

After these processing are executed or when the relevance is not detected but adding of new COA is permitted between other COAs, the input of new COA information is decided.

When the input of the new COA information is decided, the absolute time of the information based on the time-line information included in the linkage act characteristics 121 stored in this inputted COA is decided, and the existence or nonexistence of the relevance with COA inputted afterward are supervised.

Thus, the display screen information is updated by adding of the COA information, etc. (S12), and then it returns to processing of Step S3.

The operation is completed when the desired information is displayed by repeating the processing of the above-mentioned Step S6 to Step S13 (in the case of "YES" in S14).

According to this above embodiment, since the time scale 51 of the desired time unit can be selected and the electronic medical record information 52 can be displayed, various information from the long-term information over the several ten years by the display of the unit of decade to the information of the measurement value by the display of the unit of second which changes every moment can be displayed.

Furthermore, in the time scale 51 of the unit of second or the unit of minute, since the display screen information displayed can be updated with passage of time, acquiring the newest value, the change of the information during measurement can be displayed in real time.

Moreover, since a plurality of time scales 51 can be displayed with different time width, after moving rough time for the pointer P with the large time scale 51 of the time unit (for example, unit of year), the information corresponding to the desired time can be displayed by easy operation from the huge information over the several ten years by further moving the pointer P to the time which displays gradually with the time scale 51 of the small time unit (for example, the unit of month -> unit of day).

Moreover, the time-line length (horizontal direction) or the number of the display items of the electronic medical record information 52 displayed by the window area manager can be adjusted, and the information of the desired area can be displayed in the desired size.

Moreover, since the linkage characteristic information with other COAs stored in the information of the COA displayed on the electronic medical record information 52 can be displayed with the time scale 51 of the desired time unit, the dependency and exclusion relationship of COAs can be displayed intelligibly when planning the treatment.

Moreover, it is also possible to build the project information display program as which a computer is operated as an electronic medical record display device by programming the functional constitution of the display information operation unit, the display information generation unit, and the storage unit of the electronic medical record display device of the above-mentioned embodiment, and incorporating these in the concerned computer. This project information display program is available also as software published to Web on the network, etc.

Moreover, in this embodiment, although the electronic medical record display device is explained as an example of the project information display device, it is not limited to this, but it may be applied when displaying other project information, for example, the project information etc. for managing the fabricating process at the factory.

Moreover, in this embodiment, although the storage unit has the configuration provided in the electronic medical record display device, it is not limited to this, but it may provide in a device, such as a server, connected with another devices which have the display information operation unit and the display information generation unit through a network.

By composing in this way, also when the amount of information in the storage unit becomes huge, it can correspond to.

Moreover, although the case where the time unit of the time scale displayed on the display screen in this embodiment is selected from the unit of decade, the unit of year, the unit of month, the unit of day, the unit of hour, the unit of minute, or the unit of second is explained, it is not limited to these but can select according to the content which is displayed if it is the units showing time or the order, such as weekly, the unit of 1000-years, the unit of 10,000-years, the unit of 100 million-years, the unit of millisecond, the unit of microsecond, or the unit set up arbitrarily.

Moreover, although the case where the three time units of the time scale displayed on the display screen in the time unit selection unit in this embodiment are selected is explained, it is not limited to this, and it is also possible to change into other numbers of the lines, such as four or two, if needed.

For example, when displaying the time scale by the two time units, it may enable to select the display by the monthly calendar as which the date is displayed for every day of the week per month, as one form of the time scale of the time unit of the high order among these two time scales.

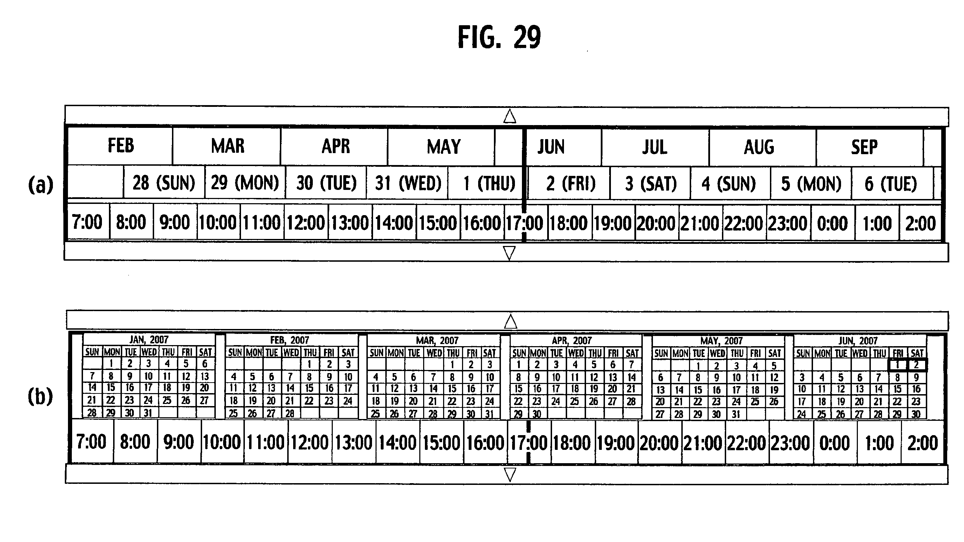

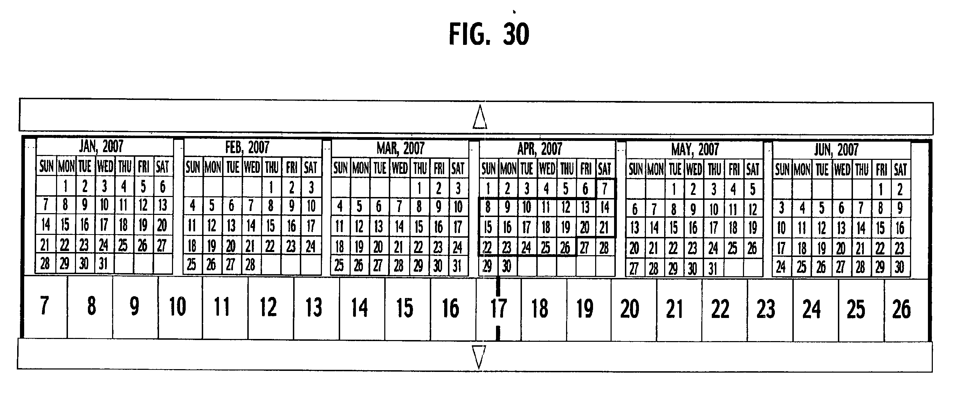

An example of the display by this monthly calendar will be explained with reference to Fig. 29 and Fig. 30.

As shown in Fig. 29(a), when the pointer P is adjusted at "17:00 on June 1 (Thursday)", and being displayed at "7:00 on June 1" to "2:00 on June 2" in the time scale of the unit of hour which is the minimum time unit, if it is selected so that the time scale of the time unit of the high order may be displayed by the monthly calendar, it is displayed as shown in Fig. 29(b), and the date part of the monthly calendar corresponding to the period currently displayed with the time scale time scale of the unit of hour which is the low order time unit is emphasized and displayed. Here, "June 1" and "June 2" are emphasized.

At this point, if the date emphasized when click operation or drag operation is performed by the user within the monthly calendar is added, the time scale of the unit of hour including the time width of the day which the width per hour of the unit of hour which is the time unit of the low order decreased and added is displayed.

The time scale of the unit of hour which is the time unit of this low order sets up the limit value of the displayed period (time number), and may set up change from the unit of hour to the unit of day when this limit value is exceeded.

For example, in Fig. 30, the low order time unit is changed and displayed on the unit of day by selecting the long period of time on "April 7" to "April 26".

When this monthly calendar is displayed, the pointer P is displayed only on the time scale of the low order time unit, and the time acting as the reference point of the display information shown with this pointer P can move the time scale by performing the click operation or the sliding operation.

Moreover, the display of the monthly calendar is operation of operating the go button (not shown) installed in the right and left, or performing sliding operation of the title part of every month of the monthly calendar, the month to display can be changed. Moreover, if operation of moving between the time-lines of the time scale which is operation of up and down for changing the time unit of the time scale mentioned above, operation for selecting the vertical button of the both ends of the time scale 51, or operation for selecting the vertical button placed around the time scale is performed, it can change to the time-line display and the function by the various modes can be used.

Moreover, when displaying the time scale in this embodiment, the time unit arranged from the upper part of the screen sequentially from the larger one, but the time unit may arrange and display from the upper part of the screen sequentially from the smaller one conversely.

Moreover, various kinds of modes set up in this embodiment can be combined with multifariousness according to the object to be used.

For example, in the conversion in the time travel scale mode from the parallel time scale mode, when the mouse or the pen is moved onto the time-line of the lowest time unit, it does not change from the parallel time scale mode to the time travel scale mode, but it may be changed on other time-lines, and when changing the display period of the information, it may set up the mode which can use operation by the slide also at the time of the click mode.

Moreover, in this embodiment, although set up change arbitrarily the window area of the display screen information displayed by clicking or dragging the window area manager, it is not limited to this. For example, the window area manager divided in the shape of 3×3 grids may be displayed, and by clicking one of these grids, it may set up so that window area can be changed into several steps.

In addition, although the electronic medical record display device for displaying the treatment plan of the electronic medical record in the medical field is explained in this embodiment, it is also possible to use the project information display device of the present invention for: (1) the relational analysis of the interaction based on the time passage about analyzing of the phenomena, such as the reaction between the numerators in natural science field; (2) display of the time in the light-year level in the field of space science, and analyzing of the relationship on the time-line of the natural phenomenon which has occurred in the space; (3) analysis of the time relation of the generated matter in the chronological table, and analysis of important histories, such as the correlation of the Western history, and the Japanese history; and (4) calendar information of the schedule management software on business management software, etc.

**Claims**

**1.** A project information display device comprising:

a storage unit configured to store calendar information, a plurality of act information and project information , wherein the calendar information is including date information, the plurality of act information is detailed information for every act performed in a project, the project information is including act information selected from the plurality of act information and execution time information for which this selected act information are performed;

a time scale generation unit configured to acquire the calendar information of a period designated as a display period from the storage unit, to generate a plurality of time scales of a different time unit;

a project information acquisition unit configured to acquire the project information from the storage unit , wherein the project information is corresponding to a time scale of the smallest time unit among a plurality of time scales generated by the time scale generation unit, and is included the execution time information within the display period unit;

a display screen information generation unit configured to generate display screen information from the plurality of time scales unit and the project information unit, wherein the time scales are generated by the time scale generation unit and the project information is acquired in the project information acquisition unit; and

a display unit configured to display the display screen information generated by the display screen information generation unit.

2. The project information display device according to claim 1, wherein
the plurality of time scales generated by the time scale generation unit are generated with the same time width, all in all, or are generated with different time width, respectively.

3. The project information display device according to claim 1 or 2, wherein
the plurality of time scales generated by the time scale generation unit include arbitrarily at least a time scale generated to unit of decade, unit of year, unit of month, unit of week, unit of day, unit of hour, unit of minute, and unit of second.

4. The project information display device according to any one of claims 1 to 3, wherein:

the plurality of time scales generated by the time scale generation unit includes a pointer showing a reference point among the calendar information;

the time scale generation unit changes the display period of the time scale by moving the pointer on the generated time scale, or moving the time scale against the fixed pointer;

in connection with this, the project information acquisition unit updates the acquired project information according to the display period of the time scale changed in the time scale generation unit; and

the display screen information generation unit updates the display screen information according to the display period of the time scale changed in the time scale generation unit, and the project information updated in the project information acquisition unit.

5. The project information display device according to claim 4, wherein
the time scale generation unit changes the display period of the time scale for an arbitrary position designated by click operation on the time scale of an arbitrary time unit in the generated time scale, or an arbitrary position designated by performing sliding operation of the time scale of an arbitrary time unit, as a position of the pointer.

6. The project information display device according to any one of claims 1 to 5, wherein:

when an arbitrary time range is selected from one of the time scale among the generated time scales, the time scale generation unit changes the display period of the plurality of time scales into all the display rectangles of the time scale of a minimum time unit among the plurality of generated time scales with the application of this selected time range;

in connection with this, the project information acquisition unit updates the acquired project information according to the display period of the time scale changed in the time scale generation unit; and

the display screen information generation unit updates the display screen information according to the display period of the time scale changed in the time scale generation unit, and the project information updated in the project information acquisition unit.

7. The project information display device according to any one of claims 1 to 6, wherein:

the act information stored by the act information storage unit includes linkage information comprising the applicable condition for the own act and other acts to have a relevance for the every performed act; and
when a new act information is added to the acquired project information, the project information acquisition unit detects mutually the act information in the project information to which the linkage information included in the act information concerned is applied, and generates output information based on a detected result.

8. The project information display device according to any one of claims 1 to 7, wherein
the display screen information generation unit includes a window area manager for generating display rectangle information for selecting a display rectangle of the display screen information to be generated, and enlarging or reducing the content of the display, and generates the display screen information from the display rectangle information generated in the window area manager, the plurality of time scales generated by the time scale generation unit, and the project information acquired in the project information acquisition unit.

9. The project information display device according to claim 8, wherein
in the display screen of the display unit, as for the display screen information, the plurality of time scales are displayed on the upper part of the display screen, the pro j ect information acquired in the project information acquisition unit is displayed on the lower part of the plurality of time scales, and the window area manager is displayed on the upper left end of the display screen.

10. The project information display device according to any one of claims 1 to 9, wherein:

the time scale generation unit updates the display period of the acquired calendar information with a predetermined interval with passage of time;
in connection with this, the project information acquisition unit updates the acquired project information according to the display period of the time scale updated in the time scale generation unit; and
the display screen information generation unit updates the display screen information according to the display period of the time scale updated in the time scale generation unit, and the project information updated in the project information acquisition unit.

11. The project information display device according to any one of claims 1 to 10, wherein
one of the plurality of time scales is a plurality of monthly calendars with which the date is arranged for every day of a week.

12. A project information display method comprising:

a storing step for storing project information composed by calendar information including date information, a plurality of act information which is detailed information for every act performed in a project, and act information selected from the plurality of act information, and execution time information for which this selected act information is performed;
a time scale generation step for acquiring the calendar information of a period designated as a display period from the calendar information stored by the storing step, and generating a plurality of time scales of the different time unit;
aproject information acquiring step for acquiring the project information corresponding to the time scale of a smallest time unit among the plurality of time scales generated by the time scale generation step, and being included the execution time information within the display period, from the project information stored by the storing step;
a display screen information generation step for generating display screen information from the plurality of time scales generated by the time scale generation step, and the project information acquired by the project information acquiring step; and
a displaying step for displaying the display screen information generated by the display screen information generation step.

13. The project information display method according to claim 12, wherein
the plurality of time scales generated by the time scale generation step are generated with the same time width all in all, or are generated with different time width, respectively.

14. The project information display method according to claim 12 or 13, wherein
the plurality of time scales generated by the time scale generation step include arbitrarily at least a time scale generated to unit of decade, unit of year, unit of month, unit of week, unit of day, unit of hour, unit of minute, and unit of second.

15. The project information display method according to any one of claims 12 to 14, wherein:

the plurality of time scales generated by the time scale generation step includes a pointer showing a reference point of the calendar information;

the time scale generation step changes the display period of the time scale by moving the pointer on the generated time scale, or moving the time scale toward the fixed pointer;

in connection with this, the project information acquiring step updates the acquired project information according to the display period of the time scale changed in the time scale generation step; and

the display screen information generation step updates the display screen information according to the display period of the time scale changed in the time scale generation step, and the project information updated in the project information acquiring step.

16. The project information display method according to claim 15, wherein

the time scale generation step changes the display period of the time scale for an arbitrary position designated by click operation on the time scale of an arbitrary time unit among the generated time scale, or an arbitrary position designated by performing sliding operation of the time scale of an arbitrary time unit, as a position of the pointer.

17. The project information display method according to any one of claims 12 to 16, wherein:

when an arbitrary time range is selected from among one of the time scale among the plurality of generated time scale, the time scale generation step changes the display period of the plurality of time scales into all the display rectangles of the time scale of a minimum time unit among the plurality of generated time scales with the application of this selected time range;

in connection with this, the project information acquiring step updates the acquired project information according to the display period of the time scale changed in the time scale generation step; and

the display screen information generation step updates the display screen information according to the display period of the time scale changed in the time scale generation step, and the project information updated in the project information acquiring step.

18. The project information display method according to any one of claims 12 to 17, wherein:

the act information stored by the storing step includes linkage information composed of the applicable condition for the own act and other acts to have a relevance for the every performed act; and

when the new act information is added to the acquired project information, the project information acquiring step detects mutually the act information in the project information to which the linkage information included in the act information concerned is applied, and generates output information based on a detected result.

19. The project information display method according to any one of claims 12 to 18 further comprising

a display rectangle selection step for selecting a display rectangle of the display screen information, wherein

the display screen information generation step generates display screen information from the display rectangle information selected by the display rectangle selection step, the plurality of time scales generated by the time scale generation step, and the project information acquired by the project information acquiring step.

20. The project information display method according to claim 19, wherein

the display screen generation step generates display screen information so that the plurality of time scales is displayed on the upper part of the display screen, the project information is displayed on the lower part of the plurality of time scales, and a window area manager for executing the display rectangle selection step is displayed on the upper left end of the display screen.

21. The project information display method according to any one of claims 12 to 20, wherein

the time scale generation step updates the display period of the acquired calendar information with a predetermined interval with passage of time;

in connection with this, the project information acquiring step updates the acquired project information according to the display period of the time scale updated in the time scale generation step; and

the display screen information generation step updates the display screen information according to the display period of the time scale updated in the time scale generation step, and the project information updated in the project information acquiring step.

22. The project information display method according to any one of claims 12 to 21, wherein

one of the plurality of time scales is a plurality of monthly calendars with which the date is arranged for every day of a week.

23. A project information display program for achieving a plurality of functions to a project information display device, the project information display program comprising:

a function for storing calendar information, a plurality of act information and project information, wherein the calendar information is including date information, a plurality of act information is detailed information for every act performed in a project, the project information is including act information selected from the plurality of act information, and execution time information for which this selected act information is performed;

a function for acquiring the calendar information of a period designated as a display period from the stored calendar information, to generate a plurality of time scales of the different time unit;

a function for acquiring the project information from the storage unit , wherein the project information is corresponding to the time scale of the smallest time unit among the plurality of generated time scales, and is included the execution time information within the display period ;

a function for generating display screen information from the plurality of generated time scales and the project information, wherein the project information is corresponding to the time information of the time scale of the acquired smallest time unit; and

a function for displaying the generated display screen information.

24. The project information display program according to claim 23, wherein
the plurality of time scales generated by the time scale generation step are generated with the same time width all in all, or are generated with different time width, respectively.

25. The project information display program according to claim 23 or 24, wherein
the plurality of time scales generated by the time scale generation step include arbitrarily at least a time scale generated to unit of decade, unit of year, unit of month, unit of week, unit of day, unit of hour, unit of minute, and unit of second.

26. The project information display program according to any one of claims 23 to 25, wherein
the generated plurality of time scales includes a pointer showing a reference point of the calendar information, and the project information display program further comprises:

a function for changing the display period of the time scale by moving the pointer on the generated time scale, or moving the time scale against the fixed pointer;

a function for updating the acquired project information according to the changed display period of the time scale; and

a function for updating the display screen information according to the changed display period of the time scale, and the updated project information.

27. The project information display program according to claim 26 further comprising
a function for changing the display period of the time scale for an arbitrary position designated by click operation on the time scale of an arbitrary time unit in the generated time scale, or an arbitrary position designated by performing sliding operation of the time scale of an arbitrary time unit, as a position of the pointer.

28. The project information display program according to any one of claims 23 to 27 further comprising:

a function for changing the display period of the plurality of time scales into all the display rectangles of the time scale of a minimum time unit among the plurality of generated time scales with the application of this selected time range when an arbitrary time range is selected from one of the time scale among the generated time scales;

in connection with this, a function for updating the acquired project information according to the changed display period of the time scale; and

a function for updating the display screen information according to the changed display period of the time scale, and the updated project information.

29. The project information display program according to any one of claims 23 to 28, wherein
the act information includes linkage information comprising the applicable condition for the own act and other acts to have a relevance for the every performed act; and
a function for detecting mutually the act information in the project information to which the linkage information included in the act information concerned is applied, and for generating output information based on a detected result, when a new act information is added to the acquired project information.

30. The project information display program according to any one of claims 23 to 29 further comprising:

a function for selecting a display rectangle of the display screen information; and
a function for generating display screen information from the display rectangle information selected by the display rectangle selection function, the generated plurality of time scales, and acquired the project information.

31. The project information display program according to claim 30 wherein
the display screen information is generated so that the plurality of time scales is displayed on the upper part of the display screen, the project information is displayed on the lower part of the plurality of time scales, and a window area manager for executing the display rectangle selection step is displayed on the upper left end of the display screen.

32. The project information display program according to any one of claims 23 to 31 further comprising:

a function for updating the display period of the acquired calendar information with a predetermined interval with passage of time;
a function, in connection with this, for updating the acquired project information according to the updated display period of the time scale; and
a function for updating the display screen information according to the updated display period of the time scale, and the updated project information.

33. The project information display program according to any one of claims 23 to 32 wherein
one of the plurality of time scales is a plurality of monthly calendars with which the date is arranged for every day of a week.

34. An electronic medical record information display device comprising:

a storage unit configured to store calendar information, a plurality of act information and medical record information, wherein the calendar information is including date information, the plurality of act information is detailed information for every act performed in a project, the medical record information is including act information selected from the plurality of act information and execution time information for which this selected act information are performed;
a time scale generation unit configured to generate a plurality of time scales of the different time unit;
a medical record information acquisition unit configured to acquire the medical record information from the storage unit, wherein the medical record information is corresponding to a time scale of the smallest time unit among a plurality of time scales generated by the time scale generation unit, and being included the execution time information within the display period;
a display screen information generation unit configured to generate display screen information from the plurality of time scales unit, and the medical record information, wherein the time scales is generated by the time scale generation unit and the plurality of time scales are acquired in the medical record information acquisition unit; and
a display unit configured to display the display screen information generated by the display screen information generation unit.

# FIG. 1

EP 2 051 201 A1

**FIG. 2**

**(1) LAYER FOR ACT INFORMATION 100**

    (i) SELF-ACT INFORMATION 110 ┬── SELF-ACT CHARACTERISTICS 111 ──┬── TITLE 111a

                                                                 ├── CLASSIFICATION 111b

                                   └── SELF-ACT LOGIC 112                   ├── RELEVANT ID 111C

                                                                └── KNOWLEDGE 111d

    (ii) LINKAGE ACT INFORMATION 120 ──┬── LINKAGE ACT CHARACTERISTICS 121

                                                       └── LINKAGE ACT LOGIC 122

**(2) LAYER FOR ACT OPERATION 200**

    LOGIC CONTROL AMONG OPERATIONS : ORDER, CONDITIONAL FUNCTION, ETC.

**(3) LAYER FOR OUTPUT 300** ──┬── STATUS 300a

                                ├── EVALUATION 300b

                                ├── OUTCOMES 300c

                                └── INTERVENTION 300d

**(4) LAYER FOR ADMINISTRATION 400** ──┬── ADMINISTRATION ID GROUP 400a

                                       ├── ADMINISTRATION TITLE 400b

                                       ├── COPYRIGHT 400c

                                       └── VERSION INFORMATION 400d

EP 2 051 201 A1

# FIG. 3

**FIG. 4**

| | | | | | | | | | | | | | | | | | | | |
|---|---|---|---|---|---|---|---|---|---|---|---|---|---|---|---|---|---|---|---|
| MONTH | JUN | | | | | | | | | | | | | | | | | | MONTH |
| DAY | 1 (THU) | | | | | | | | | | | | | | | | 2 (FRI) | | DAY |
| HOUR | 7 | 8 | 9 | 10 | 11 | 12 | 13 | 14 | 15 | 16 | 17 | 18 | 19 | 20 | 21 | 22 | 23 | 0 | 1 | 2 | HOUR |

P

EP 2 051 201 A1

# FIG. 5

EP 2 051 201 A1

# FIG. 6

DISPLAY AREA ON SCREEN 600pixel

P

(a) DISPLAY BY
UNIT OF DAY

| | 28 (SUN) | 29 (MON) | 30 (TUE) | 31 (WED) | 1 (THU) | 2 (FRI) | 3 (SAT) | 4 (SUN) | 5 (MON) | 6 (TUE) | |

(b) DISPLAY BY
UNIT OF HOUR

| 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 |

(c) DISPLAY BY
UNIT OF MINUTE

| 5 | 10 | 15 | 20 | 25 | 30 | 35 | 40 | 45 | 50 | 55 | 0 | 5 | 10 | 15 |

UNIT OF MINUTE

EP 2 051 201 A1

# FIG. 7

| DECADE | 1990s | | 2000s | | 2010s | | 2020s | | 2030s | | DECADE |
|---|---|---|---|---|---|---|---|---|---|---|---|
| YEAR | 2003 | | 2004 | | 2005 | 2006 | | 2007 | 2008 | 2009 | YEAR |
| MONTH | FEB | | MAR | | APR | MAY | | JUN | JUL | AUG | SEP | MONTH |

| | 28 (SUN) | 29 (MON) | 30 (TUE) | 31 (WED) | 1 (THU) | 2 (FRI) | 3 (SAT) | 4 (SUN) | 5 (MON) | 6 (TUE) |
|---|---|---|---|---|---|---|---|---|---|---|

| 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 |
|---|---|---|---|---|---|---|---|---|---|---|---|---|---|---|---|---|---|---|---|

UNIT OF MINUTE

UNIT OF SECOND

EP 2 051 201 A1

# FIG. 8

EP 2 051 201 A1

| 1990s | 2000s | 2010s | 2020s | 2030s |
|-------|-------|-------|-------|-------|

| YEAR | 2003 | 2004 | 2005 | 2006 | 2007 | 2008 | 2009 | YEAR |
|------|------|------|------|------|------|------|------|------|
| MONTH | FEB | MAR | APR | MAY | JUN | JUL | AUG | SEP | MONTH |

| DAY | | 28 (SUN) | 29 (MON) | 30 (TUE) | 31 (WED) | 1 (THU) | 2 (FRI) | 3 (SAT) | 4 (SUN) | 5 (MON) | 6 (TUE) | DAY |
|-----|--|----------|----------|----------|----------|---------|---------|---------|---------|---------|---------|-----|

| 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 |
|------|------|------|-------|-------|-------|-------|-------|-------|-------|-------|-------|-------|-------|-------|-------|-------|------|------|------|

UNIT OF MINUTE

UNIT OF SECOND

**FIG. 9**

| 1990s | 2000s | 2010s | 2020s | 2030s |
|---|---|---|---|---|

| 2003 | 2004 | 2005 | 2006 | 2007 | 2008 | 2009 |
|---|---|---|---|---|---|---|

MONTH / DAY / HOUR

| FEB | MAR | APR | MAY | JUN | JUL | AUG | SEP |
|---|---|---|---|---|---|---|---|

| 28 (SUN) | 29 (MON) | 30 (TUE) | 31 (WED) | 1 (THU) | 2 (FRI) | 3 (SAT) | 4 (SUN) | 5 (MON) | 6 (TUE) |
|---|---|---|---|---|---|---|---|---|---|

| 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 |
|---|---|---|---|---|---|---|---|---|---|---|---|---|---|---|---|---|---|---|---|

UNIT OF MINUTE

UNIT OF SECOND

# FIG. 10

| 1990s | 2000s | 2010s | 2020s | 2030s |
|---|---|---|---|---|

| 2003 | 2004 | 2005 | 2006 | 2007 | 2008 | 2009 |
|---|---|---|---|---|---|---|

| FEB | MAR | APR | MAY | JUN | JUL | AUG | SEP | |
|---|---|---|---|---|---|---|---|---|

| DAY | | 28 (SUN) | 29 (MON) | 30 (TUE) | 31 (WED) | 1 (THU) | 2 (FRI) | 3 (SAT) | 4 (SUN) | 5 (MON) | 6 (TUE) | | DAY |
|---|---|---|---|---|---|---|---|---|---|---|---|---|---|

| HOUR | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | HOUR |
|---|---|---|---|---|---|---|---|---|---|---|---|---|---|---|---|---|---|---|---|---|---|

MINUTE    UNIT OF MINUTE    MINUTE

UNIT OF SECOND

EP 2 051 201 A1

# FIG. 11

EP 2 051 201 A1

| 1990s | 2000s | 2010s | 2020s | 2030s |
|-------|-------|-------|-------|-------|

| 2003 | 2004 | 2005 | 2006 | 2007 | 2008 | 2009 |
|------|------|------|------|------|------|------|

| FEB | MAR | APR | MAY | JUN | JUL | AUG | SEP | |
|-----|-----|-----|-----|-----|-----|-----|-----|--|

| | 28 (SUN) | 29 (MON) | 30 (TUE) | 31 (WED) | 1 (THU) | 2 (FRI) | 3 (SAT) | 4 (SUN) | 5 (MON) | 6 (TUE) | |
|--|----------|----------|----------|----------|---------|---------|---------|---------|---------|---------|--|

| HOUR | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | HOUR |
|------|------|------|------|-------|-------|-------|-------|-------|-------|-------|-------|-------|-------|-------|-------|-------|-------|------|------|------|------|

| MINUTE | UNIT OF MINUTE | MINUTE |
|--------|----------------|--------|

| SECOND | UNIT OF SECOND | SECOND |
|--------|----------------|--------|

EP 2 051 201 A1

# FIG. 12

| | MONTH | FEB | MAR | APR | MAY | JUN | JUL | AUG | SEP | MONTH |
|---|---|---|---|---|---|---|---|---|---|---|
| DAY | | 2 (SUN) | 29 (MON) | 30 (TUE) | 31 (WED) | 1 (THU) | 2 (FRI) | 3 (SAT) | 4 (SUN) | 5 (MON) | 6 (TUE) | DAY |

HOUR  7:00  8:00  9:00  10:00  11:00  12:0  13:00  14:00  15:00  16:00  17:00  18:00  19:00  20:00  21:00  22:00  23:00  0:00  1:00  2:00  HOUR

51b  51a  51c  51  51b

51b  51a  51c  51b

EP 2 051 201 A1

# FIG. 13

# FIG. 14

START

POWERING ON AND INPUT OF PATIENT PERSONAL INFORMATION —— S1

GENERATION OF DEFAULT TIME SCALE —— S2

ACQUISITION OF ELECTRONIC MEDICAL RECORD INFORMATION CORRESPONDING TO TIME SCALE —— S3

GENERATION OF DISPLAY SCREEN INFORMATION —— S4

DISPLAY OF DISPLAY SCREEN INFORMATION —— S5

DISPLAY MODE IS CHANGED? S6
— YES
— NO

TIME UNIT IS CHANGED? S8
— YES
— NO

OPERATIONAL MODE IS CHANGED? S9
— YES
— NO

DISPLAY PERIOD IS CHANGED? S10
— YES
— NO

UPDATE OF TIME SCALE S7

WINDOW AREA IS CHANGED? S11
— YES
— NO

ELECTRONIC MEDICAL RECORD INFORMATION IS UPDATE? S13
— YES
— NO

UPDATE OF DISPLAY SCREEN INFORMATION S12

COMPLETE OF OPERATION? S14
— NO
— YES

END

# FIG. 15

ELECTRO-CARDIOGRAM

AVERAGE BLOOD PRESSURE

RESPIRATION

BLOOD OXYGEN SATURATION

EP 2 051 201 A1

# FIG. 16

(a)

P  C1  51

| MONTH | FEB | | MAR | | APR | | MAY | | JUN | | JUL | | AUG | | SEP | | MONTH |
|---|---|---|---|---|---|---|---|---|---|---|---|---|---|---|---|---|---|
| DAY | | 28 (SUN) | 29 (MON) | 30 (TUE) | 31 (WED) | 1 (THU) | 2 (FRI) | 3 (SAT) | 4 (SUN) | 5 (MON) | 6 (TUE) | | | | | | DAY |
| HOUR | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | HOUR |

(b)

P  51

| MONTH | MAR | | APR | | MAY | | JUN | | JUL | | AUG | | SEP | | OCT | | MONTH |
|---|---|---|---|---|---|---|---|---|---|---|---|---|---|---|---|---|---|
| DAY | | 1 (SAT) | 2 (SUN) | 3 (MON) | 4 (TUE) | 5 (WED) | 6 (THU) | 7 (FRI) | 8 (SAT) | 9 (SUN) | 10 (MON) | | | | | | DAY |
| HOUR | 5:00 | 6:00 | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | HOUR |

C2

(c)

P  51

| MONTH | MAR | | APR | | MAY | | JUN | | JUL | | AUG | | SEP | | OCT | | MONTH |
|---|---|---|---|---|---|---|---|---|---|---|---|---|---|---|---|---|---|
| DAY | | 3 (MON) | 4 (TUE) | 5 (WED) | 6 (THU) | 7 (FRI) | 8 (SAT) | 9 (SUN) | 10 (MON) | 11 (TUE) | 12 (WED) | | | | | | DAY |
| HOUR | 2:00 | 3:00 | 4:00 | 5:00 | 6:00 | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | HOUR |

EP 2 051 201 A1

# FIG. 17

**(a)**

| MONTH | FEB | | MAR | | APR | | MAY | | JUN | | JUL | | AUG | | SEP | | MONTH |
|---|---|---|---|---|---|---|---|---|---|---|---|---|---|---|---|---|---|
| DAY | | 28 (SUN) | 29 (MON) | | 30 (TUE) | | 31 (WED) | | 1 (THU) | | 2 (FRI) | | 3 (SAT) | | 4 (SUN) | 5 (MON) | 6 (TUE) | DAY |
| HOUR | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | HOUR |

P   C3   51

**(b)**

| MONTH | MAR | | APR | | MAY | | JUN | | JUL | | AUG | | SEP | | OCT | | MONTH |
|---|---|---|---|---|---|---|---|---|---|---|---|---|---|---|---|---|---|
| DAY | | 28 (TUE) | 29 (WED) | | 30 (THU) | | 31 (FRI) | | 1 (SAT) | | 2 (SUN) | | 3 (MON) | | 4 (TUE) | 5 (WED) | 6 (THU) | DAY |
| HOUR | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | HOUR |

P   51

C4

**(c)**

| MONTH | MAR | | APR | | MAY | | JUN | | JUL | | AUG | | SEP | | OCT | | MONTH |
|---|---|---|---|---|---|---|---|---|---|---|---|---|---|---|---|---|---|
| DAY | 28 (TUE) | 29 (WED) | 30 (THU) | 31 (FRI) | 1 (SAT) | 2 (SUN) | 3 (MON) | 4 (TUE) | 5 (WED) | 6 (THU) | 7 (FRI) | DAY |
| HOUR | 7:00 | 8:00 | 9:00 | 10:00 | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | HOUR |

P   51

EP 2 051 201 A1

FIG. 18

EP 2 051 201 A1

P

51

| MONTH | FEB | | MAR | APR | MAY | JUN | JUL | AUG | SEP | MONTH |
|---|---|---|---|---|---|---|---|---|---|---|
| DAY | | 28 (SUN) | 29 (MON) | 30 (TUE) | 31 (WED) | | 3 (SAT) | 4 (SUN) | 5 (MON) | 6 (TUE) | DAY |
| HOUR | 7 | 8 | 9 | 10 | 11 | 12 | 13 | 14 | 15 | 16 | 17 | 18 | 19 | 20 | 21 | 22 | 23 | 0 | 1 | 2 | HOUR |

FIG. 19

EP 2 051 201 A1

# FIG. 20

| MONTH | FEB | | MAR | | APR | | MAY | | JUN | | JUL | | AUG | | SEP | | MONTH |
|---|---|---|---|---|---|---|---|---|---|---|---|---|---|---|---|---|---|
| DAY | | 28 (SUN) | 29 (MON) | 30 (TUE) | 31 (WED) | 1 (THU) | 2 (FRI) | 3 (SAT) | 4 (SUN) | 5 (MON) | 6 (TUE) | | DAY |
| HOUR | 7 | 8 | 9 | 10 | 11 | 12 | 13 | 14 | 15 | | | | 20 | 21 | 22 | 23 | 0 | 1 | 2 | HOUR |

P

51

# FIG. 21

| MONTH | FEB | MAR | APR | MAY | | JUN | JUL | AUG | SEP | MONTH |
|---|---|---|---|---|---|---|---|---|---|---|
| DAY | | 28 (SUN) | 29 (MON) | 30 (TUE) | 31 (WED) | 1 (THU) | 2 (FRI) | 3 (SAT) | 4 (SUN) | 5 (MON) | 6 (TUE) | DAY |
| HOUR | 16 | | 17 | | | 18 | | 19 | | HOUR |

EP 2 051 201 A1

# FIG. 22

EP 2 051 201 A1

# FIG. 23

FIG. 24

| DECADE | 880s | 890s | 900s | 910s | 920s | 930s | 940s | 950s | 960s | 970s | 980s | 990s | 000s | 010s | 020s | 030s | 040s | 050s | 060s | 070s | 080s | 090s | 100s | 110s | DECADE |
|--------|------|------|------|------|------|------|------|------|------|------|------|------|------|------|------|------|------|------|------|------|------|------|------|------|--------|
| YEAR | 89 | 90 | 91 | 92 | 93 | 94 | 95 | 96 | 97 | 98 | 99 | 00 | 01 | 02 | 03 | 04 | 05 | 06 | 07 | 08 | 09 | 10 | 11 | 12 | YEAR |

51

P

52

| | 89 | 90 | 91 | 92 | 93 | 94 | 95 | 96 | 97 | 98 | 99 | 00 | 01 | 02 | 03 | 04 | 05 | 06 | 07 | 08 | 09 | 10 | 11 | 12 |
|---|---|---|---|---|---|---|---|---|---|---|---|---|---|---|---|---|---|---|---|---|---|---|---|---|
| TOTAL DOSE OF PREDONINE PER YEAR (g/YEAR) | | 30 | 45 | 20 | 15 | 25 | 11 | 10 | 9 | 8 | 5 | 3 | 2 | 1 | 30 | 15 | 7 | 3 | 1 | | | | | |
| TOTAL DOSE OF THE CYCLOSPORIN PER YEAR (g/YEAR) | | 30 | 30 | 30 | 25 | | 25 | 25 | 25 | 25 | 25 | 25 | 15 | 15 | 15 | 15 | 15 | 15 | 15 | | | | | |
| KIDNEY BIOPSY (TIMES / YEAR) | | | 1 | | | 1 | | 1 | | | | | | 1 | | | | 1 | | | | | | |
| TOTAL DOSE OF PULSE THERAPY (g/YEAR) | | 1 | | 1 | | | | | | 1 | 1 | | 1 | | | | | 1 | | | | | | |

## FIG. 25

| DECADE | 880s | 890s | 900s | 910s | 920s | 930s | 940s | 950s | 960s | 970s | 980s | 990s | 000s | 010s | 020s | 030s | 040s | 050s | 060s | 070s | 080s | 090s | 100s | 110s | DECADE |
|---|---|---|---|---|---|---|---|---|---|---|---|---|---|---|---|---|---|---|---|---|---|---|---|---|---|
| YEAR | 89 | 90 | 91 | 92 | 93 | 94 | 95 | 96 | 97 | 98 | 99 | 00 | 01 | 02 | 03 | 04 | 05 | 06 | 07 | 08 | 09 | 10 | 11 | 12 | YEAR |
| MONTH | FEB | MAR | APR | MAY | JUN | JUL | AUG | SEP | OCT | NOV | DEC | JAN | FEB | MAR | APR | MAY | JUN | JUL | AUG | SEP | OCT | NOV | DEC | JAN | MONTH |

P, 51, 52

TOTAL DOSE OF PREDONINE PER YEAR (g/MONTH): 3 3 3 2 2 2 2 2 2 2 2 2 2 2 2

AVERAGE URINARY PROTEIN PER DAY (g/DAY): 1, 1, 1, 2

TOTAL SERUM PROTEIN (g/DL)

TOTAL DOSE OF PULSE THERAPY (g/YEAR): 1, 1, 1, 2

# FIG. 26

P · 51

| YEAR | 89 | 90 | 91 | 92 | 93 | 94 | 95 | 96 | 97 | 98 | 99 | 00 | 01 | 02 | 03 | 04 | 05 | 06 | 07 | 08 | 09 | 10 | 11 | 12 | YEAR |
|---|---|---|---|---|---|---|---|---|---|---|---|---|---|---|---|---|---|---|---|---|---|---|---|---|---|
| MONTH | FEB | MAR | APR | MAY | JUN | JUL | AUG | SEP | OCT | NOV | DEC | JAN | FEB | MAR | APR | MAY | JUN | JUL | AUG | SEP | OCT | NOV | DEC | JAN | MONTH |
| DAY | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | DAY |

| | 89 | 90 | 91 | 92 | 93 | 94 | 95 | 96 | 97 | 98 | 99 | 00 | 01 | 02 | 03 | 04 | 05 | 06 | 07 | 08 | 09 | 10 | 11 | 12 |
|---|---|---|---|---|---|---|---|---|---|---|---|---|---|---|---|---|---|---|---|---|---|---|---|---|
| ONE-DAY TOTAL AMOUNT OF PREDONINE (mg/DAY) | | 60 | 60 | 60 | 60 | 60 | 60 | | 30 | | 30 | | 30 | | 30 | | 10 | | 5 | | | | | |
| 20% GLUCOSE INTRAVENOUS-INJECTION ONE-DAY TOTAL AMOUNT (g/DAY) | | | 8 | | 8 | | 8 | | | 4 | | | | | | 4 | | | | | | | | |
| NUMBER OF TIMES OF INHALATION (TIMES/DAY) | 3 | 3 | 3 | 2 | 2 | 2 | 2 | 2 | 2 | 2 | 2 | 2 | 2 | 2 | 2 | | | | | | | | | |
| NUMBER OF TIMES OF COLLECTION OF BLOOD (TIMES/DAY) | | 1 | | | 1 | | | | 1 | | | | | | 2 | | | | | | | | | |

~52

EP 2 051 201 A1

FIG. 27

| MONTH | FEB | MAR | APR | MAY | JUN | JUL | AUG | SEP | OCT | NOV | DEC | JAN | MONTH |
|---|---|---|---|---|---|---|---|---|---|---|---|---|---|
| DAY | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | DAY |
| HOUR | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | 3:00 | 4:00 | 5:00 | 6:00 | 7:00 | 8:00 | 9:00 | 10:00 | HOUR |

P

51

52

PHYSIOLOGICAL SALINE INTRAVENOUS DRIP (mL/HOUR)

60mL/HOUR

30mL/HOUR

20% GLUCOSE INTRAVENOUS INJECTION

INHALATION

COLLECTION OF BLOOD

48

# FIG. 28

| DAY | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | DAY |
|---|---|---|---|---|---|---|---|---|---|---|---|---|---|---|---|---|---|---|---|---|---|---|---|---|---|
| HOUR | 11:00 | 12:00 | 13:00 | 14:00 | 15:00 | 16:00 | 17:00 | 18:00 | 19:00 | 20:00 | 21:00 | 22:00 | 23:00 | 0:00 | 1:00 | 2:00 | 3:00 | 4:00 | 5:00 | 6:00 | 7:00 | 8:00 | 9:00 | 10:00 | HOUR |

MINUTE | 50 | 0 | 10 | 20 UNIT OF MINUTE 30 | 40 | 50 | 0 | MINUTE

**DOPAMINE μg/kg/MINUTE**
- 5
- 3
- 5
- 1
- 5
- 1.5

**RESPIRATOR (RESPIRATORY TIMES/MINUTE)**
- 30
- 25

**XYLOCAINE INTRAVENOUS INJECTION (mg/HOUR)**
- 50
- 50

**MEILLON INTRAVENOUS INJECTION (mL/HOUR)**
- 20
- 20
- 20

FIG. 29

EP 2 051 201 A1

# FIG. 30

### JAN, 2007

| SUN | MON | TUE | WED | THU | FRI | SAT |
|---|---|---|---|---|---|---|
| | 1 | 2 | 3 | 4 | 5 | 6 |
| 7 | 8 | 9 | 10 | 11 | 12 | 13 |
| 14 | 15 | 16 | 17 | 18 | 19 | 20 |
| 21 | 22 | 23 | 24 | 25 | 26 | 27 |
| 28 | 29 | 30 | 31 | | | |

### FEB, 2007

| SUN | MON | TUE | WED | THU | FRI | SAT |
|---|---|---|---|---|---|---|
| | | | | 1 | 2 | 3 |
| 4 | 5 | 6 | 7 | 8 | 9 | 10 |
| 11 | 12 | 13 | 14 | 15 | 16 | 17 |
| 18 | 19 | 20 | 21 | 22 | 23 | 24 |
| 25 | 26 | 27 | 28 | | | |

### MAR, 2007

| SUN | MON | TUE | WED | THU | FRI | SAT |
|---|---|---|---|---|---|---|
| | | | | 1 | 2 | 3 |
| 4 | 5 | 6 | 7 | 8 | 9 | 10 |
| 11 | 12 | 13 | 14 | 15 | 16 | 17 |
| 18 | 19 | 20 | 21 | 22 | 23 | 24 |
| 25 | 26 | 27 | 28 | 29 | 30 | 31 |

### APR, 2007

| SUN | MON | TUE | WED | THU | FRI | SAT |
|---|---|---|---|---|---|---|
| 1 | 2 | 3 | 4 | 5 | 6 | 7 |
| 8 | 9 | 10 | 11 | 12 | 13 | 14 |
| 15 | 16 | 17 | 18 | 19 | 20 | 21 |
| 22 | 23 | 24 | 25 | 26 | 27 | 28 |
| 29 | 30 | | | | | |

### MAY, 2007

| SUN | MON | TUE | WED | THU | FRI | SAT |
|---|---|---|---|---|---|---|
| | | 1 | 2 | 3 | 4 | 5 |
| 6 | 7 | 8 | 9 | 10 | 11 | 12 |
| 13 | 14 | 15 | 16 | 17 | 18 | 19 |
| 20 | 21 | 22 | 23 | 24 | 25 | 26 |
| 27 | 28 | 29 | 30 | 31 | | |

### JUN, 2007

| SUN | MON | TUE | WED | THU | FRI | SAT |
|---|---|---|---|---|---|---|
| | | | | | 1 | 2 |
| 3 | 4 | 5 | 6 | 7 | 8 | 9 |
| 10 | 11 | 12 | 13 | 14 | 15 | 16 |
| 17 | 18 | 19 | 20 | 21 | 22 | 23 |
| 24 | 25 | 26 | 27 | 28 | 29 | 30 |

| 7 | 8 | 9 | 10 | 11 | 12 | 13 | 14 | 15 | 16 | 17 | 18 | 19 | 20 | 21 | 22 | 23 | 24 | 25 | 26 |
|---|---|---|---|---|---|---|---|---|---|---|---|---|---|---|---|---|---|---|---|

**INTERNATIONAL SEARCH REPORT**

| International application No. |
| --- |
| PCT/JP2007/064559 |

A. CLASSIFICATION OF SUBJECT MATTER
*G06Q50/00*(2006.01)i, *G06Q10/00*(2006.01)i

According to International Patent Classification (IPC) or to both national classification and IPC

B. FIELDS SEARCHED

Minimum documentation searched (classification system followed by classification symbols)
G06Q50/00, G06Q10/00

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| Y | JP 2003-228587 A  (Nikon Corp.),<br>15 August, 2003 (15.08.03),<br>Par. Nos. [0001] to [0038]; Figs. 15 to 17<br>(Family: none) | 1-34 |
| Y | JP 2004-102869 A  (Shimadzu Corp.),<br>02 April, 2004 (02.04.04),<br>Full text; all drawings<br>& US 2004/0093252 A1 | 1-34 |
| Y | JP 2004-38898 A  (Yugen Kaisha Sugiura<br>Gijutsushi Jimusho),<br>05 February, 2004 (05.02.04),<br>Full text; all drawings<br>(Family: none) | 2-11,13-22,<br>24-33 |

☒ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    22 August, 2007 (22.08.07) | Date of mailing of the international search report<br>    04 September, 2007 (04.09.07) |
| --- | --- |
| Name and mailing address of the ISA/<br>    Japanese Patent Office | Authorized officer |
| Facsimile No. | Telephone No. |

Form PCT/ISA/210 (second sheet) (April 2005)

**INTERNATIONAL SEARCH REPORT**

| International application No. |
| --- |
| PCT/JP2007/064559 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | Hideo AOYAGI, "Online Soft Scramble", Mac Fan, Vol.13, No.12, 01 December, 2005 (01.12.05), page 179 | 4-11,15-22, 26-33 |
| Y | JP 11-328209 A  (Canon Inc.), 30 November, 1999 (30.11.99), Par. Nos. [0030] to [0042], [0048] to [0049] (Family: none) | 6-11,17-22, 28-33 |
| Y | JP 2002-297759 A  (Sanyo Electric Co., Ltd.), 11 October, 2002 (11.10.02), Par. Nos. [0001] to [0028] (Family: none) | 7-11,18-22, 29-33 |
| Y | JP 5-216972 A  (Fujitsu Ltd.), 27 August, 1993 (27.08.93), Par. Nos. [0001] to [0003]; Fig. 8 (Family: none) | 8-11,19-22, 30-33 |
| Y | JP 2003-339651 A  (Denso Corp.), 02 December, 2003 (02.12.03), Par. Nos. [0001] to [0009], [0072] to [0080]; Fig. 15 (Family: none) | 10-11,21-22, 32-33 |

Form PCT/ISA/210 (continuation of second sheet) (April 2005)

**EP 2 051 201 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003108661 A
- JP 2706645 B
- JP 2815346 B
- JP H11212700 B